(19) Europäisches Patentamt / European Patent Office / Office européen des brevets

(11) **EP 3 570 908 B1**

(12) **EUROPÄISCHE PATENTSCHRIFT**

(45) Veröffentlichungstag und Bekanntmachung des Hinweises auf die Patenterteilung:
**22.11.2023 Patentblatt 2023/47**

(21) Anmeldenummer: **18701588.8**

(22) Anmeldetag: **11.01.2018**

(51) Internationale Patentklassifikation (IPC):
**A61M 5/142** (2006.01) **A61M 5/158** (2006.01)

(52) Gemeinsame Patentklassifikation (CPC):
**A61M 5/14248;** A61M 2005/14252;
A61M 2005/1585

(86) Internationale Anmeldenummer:
**PCT/IB2018/050166**

(87) Internationale Veröffentlichungsnummer:
**WO 2018/134708 (26.07.2018 Gazette 2018/30)**

(54) **KANÜLENINSERTIONSMECHANISMUS FÜR EIN PATCH GERÄT**

CANNULA INSERTION MECHANISM FOR A PATCH DEVICE

MÉCANISME D'INSERTION DE CANULES POUR APPAREIL DE TYPE PATCH

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **19.01.2017 CH 622017**
**23.02.2017 CH 2082017**

(43) Veröffentlichungstag der Anmeldung:
**27.11.2019 Patentblatt 2019/48**

(73) Patentinhaber: TecMed AG
**3400 Burgdorf (CH)**

(72) Erfinder:
• **STAUB, Seline**
**8604 Volketswil (CH)**
• **STREIT, Ursina**
**3322 Schönbühl (CH)**

(74) Vertreter: **Meier Obertüfer, Jürg et al**
**Ypsomed AG**
**Brunnmattstrasse 6**
**3401 Burgdorf (CH)**

(56) Entgegenhaltungen:
**WO-A2-2013/153041 WO-A2-2016/145094**
**US-A1- 2015 174 317**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

**Technisches Gebiet**

Die Erfindung betrifft Injektions- und Infusionsgeräte, insbesondere Injektions- und Infusionsgeräte, welche zur Verabreichung einer Substanz direkt auf die Haut der benutzenden Person aufgebracht, insbesondere geklebt werden, welche auch als Patch Geräte bekannt sind. Im Speziellen betrifft die Erfindung Mechanismen zur Insertion von Kanülen, insbesondere sogenannte Softkanülen, welche zum Beispiel aus Fluorpolymeren (wie Polytetrafluorethylen) oder eigenschaftsähnlichen Werkstoffen gefertigt sind.

**Allgemeine Beschreibung**

Ein Verabreichungsgerät (auch Verabreichungsvorrichtung genannt) für fluide Produkte, insbesondere eine Infusionspumpe oder ein Injektionsgerät, im speziellen eine Patch Infusionspumpe oder ein Patch Injektor, kann grundsätzlich für die Verabreichung von verschiedensten Medikamenten geeignet sein, sofern das Medikament eine Konsistenz aufweist, welche mit der Infusionspumpe oder dem Injektionsgerät ausschüttbar ist. Die erwähnte Konsistenz, damit gemeint ist zum Beispiel die Viskosität, kann es jedoch erforderlich machen, die Konstruktion des Injektionsgerätes oder der Infusionspumpe zu optimieren, um der benutzenden Person die Verabreichung des Medikamentes so angenehm wie möglich zu machen. Eine Möglichkeit für eine solche Optimierung ist die Verwendung von sogenannten Patch Geräten (Patch Infusionspumpe, insbesondere bei Behandlung von Diabetes mit Insulin, oder Patch Injektoren, insbesondere zur Verabreichung von Antikörper-Formulierungen mit hoher Viskosität). Die Geräte werden mittels Pflaster direkt auf den Körper der benutzenden Person aufgeklebt und müssen sodann nicht mehr manuell gehalten oder zum Beispiel in einem Halfter verstaut werden.

Der Begriff "Medikament" umfasst hier jede fliessfähige medizinische Formulierung, welche geeignet ist zur kontrollierten Verabreichung durch ein Mittel, wie z. B. eine Kanüle oder Hohlnadel, hindurch, beispielsweise umfassend eine Flüssigkeit, eine Lösung, ein Gel, eine Emulsion oder eine feine Suspension, welche(s) einen oder mehrere medizinische Wirkstoffe enthält. "Medikament" kann eine Zusammensetzung mit einem einzigen Wirkstoff oder eine vorgemischte oder co-formulierte Zusammensetzung mit mehreren Wirkstoffen aus einem einzelnen Behälter sein. Medikament umfasst Arzneien wie Peptide (z.B. Insuline, Insulin enthaltende Medikamente, GLP-1 enthaltende sowie abgeleitete oder analoge Zubereitungen), Proteine und Hormone, biologisch gewonnene oder aktive Wirkstoffe, Wirkstoffe auf Basis von Hormonen oder Genen, Nährformulierungen, Enzyme und weitere Substanzen sowohl in fester (suspendierter) oder flüssiger Form aber auch Polysaccharide, Vakzine, DNS oder RNS oder Oligonukleotide, Antikörper oder Teile von Antikörpern sowie geeignete Basis-, Hilfs- und Trägerstoffe.

Patch Geräte im Sinne der vorliegenden Anmeldung, also Patch Infusionspumpen oder Patch Injektoren, umfassen häufig sogenannte (Kanülen-) Insertionsmechanismen, welche dazu dienen eine Kanüle ins Gewebe der benutzenden Person einzuführen. Einige dieser Patch Geräte verwenden zur Verabreichung des fluiden Medikaments weiche Infusionskanülen, welche aus Kunststoff, insbesondere biokompatiblen Fluorpolymeren hergestellt sind. Dies hat den Vorteil, dass die weiche Infusionskanüle bei Bewegung des umliegenden Gewebes der benutzenden Person der benutzenden Person potentiell weniger Schmerzen verursacht als eine Infusionskanüle aus einem steifen Material wie Stahl. Ein weiterer Vorteil der weichen Infusionskanülen ist, dass es bei weichen Kanülen zu keinen Brüchen kommen kann. Um eine weiche Infusionskanüle in das Gewebe überhaupt einführen zu können, wird eine Insertionskanüle (oder eine Insertionsnadel) aus einen steifen Material wie Stahl verwendet. Diese Insertionskanüle ist vor dem Einführen der Infusionskanüle durch die Infusionskanüle hindurch geführt und steht über das offene Ende der Infusionskanüle hinaus. Die Insertionskanüle wird beim Einführen zusammen mit der Infusionskanüle ins Gewebe eingestochen. Hat die Infusionskanüle ihre Endposition im Gewebe erreicht, so wird die Infusionskanüle in dieser Position fixiert und die Insertionskanüle wird aus dem Gewebe zurückgezogen. Im Gewebe verbleibt sodann nur die weiche Infusionskanüle.

Bei einigen Insertionsmechanismen ist das innere Lumen der Insertionskanüle Teil des Fluidpfades, über welchen das fluide Medikament verabreicht wird. In diesem Fall wird die Insertionskanüle beim oben beschriebenen Rückzug nicht vollständig aus der Infusionskanüle herausgezogen, so dass das zu verabreichende fluide Medikament von der Insertionskanüle in die Infusionskanüle geleitet wird und von dort über eine oder mehrere Öffnungen der Infusionskanüle ins Gewebe der benutzenden Person gelangt.

Aus der US 7128727 B2 ist eine Patch Infusionspumpe 10 mit einem Insertionsmechanismus bekannt, mit welchem eine weiche Infusionskanüle 38 mit Hilfe einer Insertionskanüle 62 ins Gewebe eingebracht wird. Der beschriebene Mechanismus verwendet für die Insertionsbewegung der Infusionskanüle und den anschliessenden Rückzug der Insertionskanüle separate Federn 70 resp. 82, also mindestens eine Feder 70, welche die Energie für die Insertionsbewegung liefert und mindestens eine Feder 82, welche die Energie für die Rückzugsbewegung liefert.

Aus der US 2014142508 A1 ist eine Patch Infusionspumpe 100 mit Insertionsmechanismus bekannt, mit welchem eine weiche Infusionskanüle 176 mit Hilfe einer Insertionskanüle 174 ins Gewebe eingebracht wird. Der Antrieb des Insertionsmechanismus wird dabei mit einer Torsionsfeder 181 realisiert. Hierbei ist die Antriebsfeder 181 über einen Armsystem 183a, 183b mit einem Schlitten 184 verbunden. Die beiden Teilarme 183a und 183b sind über ein Gelenk mit einander verbunden, so dass die beiden Teilarme 183a, 183b in einer Ebene relativ zu einander verdrehbar sind. Der offenbarte Insertionsmechanismus ermöglicht die Ausführung der Insertionsbewegung und der Rückzugsbewegung mit einer Feder 181.

Aus der WO2016/145094A2 offenbart eine Medikamentenverabreichungspumpe mit einem Insertionsmechanismus 200. Der Insertionsmechanismus umfasst eine Torsionsfeder 210, welche zum Einstechen einer Nadel in ein Ziel teilentspannt werden kann. Nach erfolgter Verabreichung des Medikamentes kann die Torsionsfeder 210 weiter entspannt werden, wodurch die Nadel zurückgezogen wird.

Es ist eine Aufgabe der Erfindung Antriebe für alternative Kanüleninsertionsmechanismen sowie alternative Kanülen-Insertionsmechanismen für Verabreichungsgeräte, insbesondere Patch Geräte wie Patch Infusions-pumpen oder Patch Injektoren, bereitzustellen, welche kostengünstig herzustellen sind und gleichzeitig eine hohe Zuverlässigkeit aufweisen.

Die Aufgabe wird erfindungsgemäss durch den unabhängigen Anspruch 1 gelöst, vorteilhafte Weiterentwicklungen ergeben sich aus den abhängigen Ansprüchen, der Beschreibung und den Figuren.

In der folgenden Beschreibung werden die Begriffe distal und proximal in Bezug auf Positions- und Richtungsangaben verwendet. Distal meint aus bezüglich des Fluidpfades des Verabreichungsgerätes zur benutzenden Person hin (in Verabreichungs- oder Ausschüttrichtung) und proximal entsprechend das Umgekehrte. So handelt es sich zum Beispiel bei der Bewegung einer Infusionskanüle, welche in das Gewebe der benutzenden Person eingeführt wird um eine Bewegung in distale Richtung, im Folgenden auch Insertionsrichtung genannt. Die Rückzugsbewegung einer Insertionskanüle zurück ins Gehäuse einer Verabreichungsvorrichtung somit eine Bewegung in die proximale Richtung.

Ein Aspekt der Erfindung umfasst einen Kanüleninsertionsmechanismus für ein Patch Gerät, insbesondere eine Patch Infusionspumpe oder einen Patch Injektor. In der folgenden Beschreibung wird insbesondere auf Insertionsmechanismen für Patch Infusionspumpen eingegangen, welche aber in derselben Form auch für Patch Injektoren verwendet werden können, oder auch in anderen Verabreichungsgeräten zum Einsatz gelangen könnten ohne von der Erfindung abzuweichen.

In einem Aspekt der Erfindung umfasst die Patch Infusionspumpe ein Gehäuse. Das Gehäuse kann ein- oder mehrteilig aufgebaut sein und umfasst eine sogenannte Basis, welche den Bereich des Gehäuses darstellt, welcher über Pflaster auf der Haut der benutzenden Person angeordnet ist. Die Basis kann selber mehrteilig ausgestaltet sein und kann auch Einsätze, wie insbesondere Führungselemente umfassen, welche bei der Montage an der Basis angebracht werden. Alternativ kann die Basis auch als Einsatz für das Gehäuse ausgeführt sein.

In einem Aspekt der Erfindung umfasst die Patch Infusionspumpe einen Insertionsmechanismus zum Platzieren einer flexiblen oder weichen Infusionskanüle in Gewebe der benutzenden Person. Die flexible oder weiche Infusionskanüle wird dabei mit Hilfe einer Insertionskanüle in das Gewebe eingeführt.

In einem Aspekt der Erfindung umfasst der Insertionsmechanismus der Patch Infusionspumpe ein Zahnrad, welches durch eine Energiequelle in Bewegung, insbesondere Rotation, versetzt werden kann, und welches insbesondere rotierbar jedoch nicht verschiebbar an der Basis gelagert ist.

In einem Aspekt der Erfindung umfasst der Insertionsmechanismus einen Schlitten, an welchem die Insertionskanüle fest angeordnet ist, wobei der Schlitten operativ mit dem Zahnrad gekoppelt ist, so dass eine Bewegung, insbesondere Rotation des Zahnrads eine Bewegung des Schlittens und damit der Insertionskanüle zur Folge haben kann. Im Ausgangszustand über die Insertionskanüle gezogen ist die Infusionskanüle, welche im Rahmen des Insertionsprozesses mit Hilfe der Insertionskanüle im Gewebe der benutzenden Person deponiert wird. Nach der Deponierung wird die Insertionskanüle durch die Infusionskanüle hindurch aus dem Gewebe zurückgezogen, wobei die Lumen der beiden Kanülen miteinander verbunden bleiben, so dass das Medikament über die Insertionskanüle in die Infusionskanüle und folgend ins Gewebe der benutzenden Person geleitet werden kann.

In einem Aspekt der Erfindung umfasst der Insertionsmechanismus der Patch Infusionspumpe eine Führungskulisse (vorteilhaft mit einer oder mehreren Teilkulissen), entlang welcher das Zahnrad über Zahnungen oder Stiftanordnungen geführt werden kann, wobei eine Rotation des Zahnrades über die Führung entlang der Zahnungen oder Stiftanordnungen in eine Translation der Zahnradachse relativ zur Führungskulisse transformiert werden kann.

In einem Aspekt der Erfindung sind mehrere Zahnungen und Stiftanordnungen entlang der Führungskulisse so angeordnet, dass die Zahnradachse zeitlich gestaffelt in verschiedene Richtungen relativ zur Führungskulisse bewegbar ist.

In einem Aspekt der Erfindung ist das Zahnrad an seinem Umfang nur teilweise mit Zähnen besetzt, wobei es in zwei Sektoren unterteilt werden kann, in einen ersten Sektor, welcher gezahnt ist und einen zweiten Sektor, in welchem keine Zähne vorhanden sind. In alternativen Ausgestaltungen könnte das Zahnrad auch mehr als zwei Sektoren aufweisen, wobei gezahnte und ungezahnte Sektoren sich abwechseln.

In einem Aspekt der Erfindung umfasst die Führungskulisse eine erste gezahnte Teilkulisse und eine zweite gezahnte Teilkulisse, wobei die erste und die zweite Teilkulisse einander gegenüber liegen (antiparallel oder gespiegelt) und wobei die Zähne des ersten Sektors des Zahnrades zuerst an der Zahnung der ersten Teilkulisse abrollen und bei weiterer Rotation des Zahnrades in dieselbe Richtung sodann an der zweiten Teilkulisse abrollen und wobei die Zähne des ersten Sektors entweder in Eingriff mit der ersten Teilkulisse oder der zweiten Teilkulisse sind, nicht jedoch in Eingriff mit beiden gleichzeitig sind. Dieser sequentielle Eingriff der Zähne hat in diesem Aspekt der Erfindung zur Folge, dass sich die Führungskulisse relativ zur Zahnradachse während des Eingriffs der Zähne des ersten Sektors in die Zahnung der ersten Teilkulisse entlang der ersten Teilkulisse in eine erste Richtung bewegt und folgend beim Eingriff der Zähne des ersten Sektors in die Zahnung der zweiten Teilkulisse entlang der zweiten Teilkulisse in eine zweite Richtung bewegt, welche der ersten Richtung entgegengesetzt ist, so dass eine Hin- und Herbewegung zwischen Führungskulisse und Zahnradachse entsteht. Fakultativ kann zwischen dem Eingriff der Zähne des ersten Sektors mit der Zahnung der ersten Teilkulisse und dem Eingriff der Zähne des ersten Sektors in die Zahnung der zweiten Teilkulisse ein Phase bestehen, in welcher die Zähne des ersten Sektor nicht in Eingriff mit einer Zahnung sind, so dass das Zahnrad rotieren kann, ohne dass sich die Zahnradachse relativ zur Führungskulisse verschiebt.

In einem Teilaspekt des vorhergehenden Aspekts der Erfindung ist der maximale Winkel, welchen den ersten Sektor des Zahnrades umfasst, wie folgt definiert:

$$maximaler\ Winkel_{erster\ Sektor}$$
$$\approx 180° - ((Winkel\ pro\ Zahn)$$
$$* Gesamtzahl\ der\ Zähne\ in\ gleichzeitigem\ Eingriff\ mit\ einer\ Teilkulisse)$$

In einem weiteren Teilaspekt des vorhergehenden Aspektes der Erfindung entspricht die relative Bewegung zwischen der ersten Teilkulisse und dem Zahnrad, während das Zahnrad an der ersten Teilkulisse abrollt und die Zähne des ersten Sektors in Eingriff mit der Zahnung der ersten Teilkulisse sind, dem Weg der Insertionskanüle in beim Einführen der Infusionskanüle ins Gewebe.

In alternativen Ausgestaltungen kann das Zahnrad ein Reibrad sein, wobei die Teilkulissen dann anstatt Zahnungen oder Stiftanordnungen entsprechende Reibflächen umfassen entlang derer das Reibrad sich kraftschlüssig bewegen kann.

In einem Aspekt der Erfindung umfasst der Kanüleninsertionsmechanismus ein drehfest und koaxial am Zahnrad angeordnetes Einleitrad, insbesondere ein Einleitzahnrad. Dieses Einleitrad dient dazu, Bewegungsenergie von einer Energiequelle auf das Zahnrad zu übertragen, wobei das Zahnrad insbesondere in Rotation versetzt wird. Das Einleitrad kann zusammen mit dem Rad als ein Teil konstruiert und hergestellt sein, alternativ können Zahnrad und Einleitrad als zwei separate Teile hergestellt sein und bei der Montage fest (i. e. unbeweglich zueinander) miteinander verbunden werden, zum Beispiel über eine gemeinsame Achse oder durch Fügetechniken wie Kleben oder Schweissen.

In einem Aspekt der Erfindung umfasst der Kanüleninsertionsmechanismus ein am Gehäuse, insbesondere an der Basis, geführtes längliches Übertragungselement, welches in Kontakt mit dem Einleitrad gebracht werden kann, insbesondere so, dass Energie übertragen werden kann. Durch ein relatives Abrollen des Einleitrades am Übertragungselement kann die lineare Relativbewegung zwischen Einleitrad und Übertragungselement in eine Rotation des Einleitrades und damit eine Rotation des Zahnrades übersetzt werden. Der Kontakt zwischen Übertragungselement kann form- oder kraftschlüssig sein. In einer Variante ist das Einleitrad rotierbar, aber nicht verschiebbar an der Basis gelagert und das Übertragungselement lässt sich insbesondere linear zur Basis und damit zum Einleitrad verschieben. Hierdurch ergibt sich ein Antrieb für den Kanüleninsertionsmechanismus, bei welchem eine Linearbewegung in eine Rotationsbewegung übersetzt wird. Das Übertragungselement kann beispielhaft durch eine Antriebsfeder als Energiequelle verschoben werden. Bei der Antriebsfeder kann es sich um eine Druckfeder handeln, welche bei Freisetzen von Energie expandiert. In einer Variante kann die Antriebsfeder eine Zugfeder sein, welche bei Freisetzung von Energie kontrahiert. In einer Ausgestaltung kann für die Verschiebung des Übertragungselementes ein hydraulischer oder pneumatischer Antrieb (Zylinder/Kolben) eingesetzt werden. In einer weiteren Ausgestaltung kann für die Verschiebung des Übertragungselementes auch ein Elektroantrieb gelangen, zum Beispiel ein Elektromotor, welcher über ein Zahnrad oder ein Schneckenrad mit dem Übertragungselement verbunden wird, ein Linearmotor, insbesondere mit Piezoschwingantrieb, oder ein Solenoidantrieb,. Beim Elektromotor kann es sich um einen DC-Motor oder auch um einen Schrittmotor handeln, wobei dem Fachmann weitere Varianten geläufig sind. In einer weiteren Alternative könnten auch sich anziehende oder abstossende Magnete für den Antrieb verwendet werden. Vorteilhaft den beschriebenen elektrischen Antrieben ist, dass diese selbsthemmend ausgestaltet sein können, das heisst, es braucht keine eigentlichen Vorrichtungen, welche den Kanüleninsertionsmechanismus im Ausgangszustand oder Endzustand festhalten. Diese Selbsthemmung kann zum Beispiel über den inneren Widerstand (Rastmoment) eines Elektromotors verwirklicht sein, wenn dieser nicht eingeschaltet ist, zum Beispiel bei DC Motoren mit Permanentmagnet. Alternativ kann diese Selbsthemmung auch mittels eines Schneckenrades erzielt werden, welches Motor und Übertagungselement koppelt.

In einem Aspekt der Erfindung ist das Einleitrad als Einleitzahnrad und das Übertragungselement als Zahnstange ausgestaltet. In diesem Aspekt ist die Zahnstange linear an der Basis (linear) geführt und das Einleitzahnrad mit dem Zahnrad rotierbar an der Basis gelagert. Die Zahnstange weist eine Zahnung entlang einer Längsdimension auf, welche in Eingriff mit Zähnen des Einleitzahnrades bringbar sind, wodurch eine Bewegung der Zahnstange in eine Rotation von Einleitzahnrad und Zahnrad umwandelbar ist, wobei die Zahnstange durch Energie aus einer Feder oder einem elektrischen Antriebe in Bewegung versetzt werden kann.

In einem Aspekt der Erfindung werden das Einleitrad und damit auch das Zahnrad durch einen elektrischen Antrieb in Rotation versetzt, wobei die Zahnstange weggelassen wird, und wobei in diesem Aspekt Einleitrad und Zahnrad vorteilhaft rotier-, aber nicht verschiebbar an der Basis oder dem Gehäuse gelagert sind. Dabei kann das Einleitrad insbesondere als Kegelrad, speziell als Kegelzahnrad konstruiert sein, um das Einleiten der Energie effizient und platzsparend gestalten zu können. Das Einleitrad kann dabei durch einen Elektromotor, welcher zum Beispiel über ein Getriebe und ein weiteres Kegelrad, welches als Übertragungselement dient, und das fest an der vom Motor angetriebenen Achse angeordnet ist, mit dem Einleitrad verbunden wird, angetrieben werden, wobei das weitere Kegelrad in dieser Ausgestaltung die Funktion des Übertragungselementes übernimmt.

In einem Aspekt der Erfindung kann das Übertragungselement eine Kette, eine Saite oder ein Riemen sein, welche das Einleitrad mit dem elektrischen Antrieb koppelt.

Weitere Aspekte der Erfindung werden folgend wieder gegeben:

In einem Aspekt umfasst die Erfindung einen Kanüleninsertionsmechanismus für ein Patch Gerät mit

- einem Gehäuse umfassend eine Basis, welche auch als Basis für das Patch Gerät dienen kann und welche direkt oder indirekt auf der Haut der benutzenden Person anbringbar ist,
- mindestens einer Führungsbahn, welche fest am Gehäuse angebracht ist und zumindest einen Teil eines Insertionspfads definiert,
- einem Schlitten, welcher in der mindestens einen Führungsbahn in und gegen eine Insertionsrichtung entlang der Führungsbahn verschiebbar gelagert ist,
- einer Stahlkanüle, welche fest am Schlitten angebracht ist und in etwa parallel zur Richtung der Verschiebung vom Schlitten abragt,
- einer Antriebsfeder, insbesondere einer Torsionsfeder, deren Achse in etwa senkrecht zur mindestens einen Führungsbahn steht und mit einem ersten Ende der Antriebsfeder fest an der Basis oder dem Gehäuse anbringbar ist,
- einem Zahnrad, welches fest an einem zweiten Ende der Antriebsfeder anbringbar ist, wobei dessen Rotationsachse mit der Achse der Antriebsfeder zusammenfällt,
  wobei die Zahnung des Zahnrades einen ersten Sektor des Zahnrades umfasst, und wobei ein zweiter Sektor des Zahnrades keine Zahnung umfasst,
- einer in sich geschlossenen Führungskulisse, welche fest am Schlitten angeordnet ist, welche in einem Abstand zur mindestens einen Führungsbahn verläuft, wobei sich der Abstand aus den geometrischen Dimensionen von Schlitten, Antriebsfeder und Zahnrad ergibt, und

  wobei die Führungskulisse mindestens eine Teilkulisse umfasst, welche gleichlaufend zur Führungsbahn verläuft, wobei die mindestens eine Teilkulisse jeweils eine Zahnung oder Stiftanordnung umfasst, welche abhängig von der rotativen Orientierung des Zahnrades, mit den Zähnen des ersten Sektors in Eingriff bringbar ist,
  wobei das Zahnrad durch Freisetzung von Energie aus der Antriebsfeder in eine Rotationsbewegung versetzt wird und dadurch der Schlitten mit der Stahlkanüle durch Eingriff der Zähne des Zahnrades mit der Zahnung oder Stiftanordnung der mindestens einen Teilkulisse in eine Bewegung entlang der mindestens einen Führungsbahn versetzbar ist.

In einem Aspekt umfasst die Erfindung einen Kanüleninsertionsmechanismus für ein Patch Gerät mit einer Kanüle aus weichem Kunststoff, insbesondere PTFE, welche über die Stahlkanüle ziehbar gestaltet ist.

In einem Aspekt umfasst die Erfindung einen Kanüleninsertionsmechanismus für ein Patch Gerät, wobei die Antriebsfeder eine Torsionsfeder ist und eine helikale Form hat.

In einem Aspekt umfasst die Erfindung einen Kanüleninsertionsmechanismus für ein Patch Gerät, wobei die Antriebsfeder eine Torsionsfeder ist, welche die Form einer Spiralfeder aufweist.

In einem Aspekt umfasst die Erfindung einen Kanüleninsertionsmechanismus für ein Patch Gerät, wobei das Zahnrad einen zweiten Sektor umfasst, in welchem keine Zahnung vorhanden ist.

In einem Aspekt umfasst die Erfindung einen Kanüleninsertionsmechanismus für ein Patch Gerät, wobei die Führungskulisse zwei Teilkulissen mit Zahnung umfasst, wobei die zwei Teilkulissen in derselben Ebene liegen und die Zahnungen in einem Abstand einander gegenüberliegen, und wobei der Abstand der zwei Teilkulissen zueinander durch den Durchmesser des Zahnrades ohne dessen Zähne bestimmt wird, insbesondere durch den Teilkreisdurchmesser des Zahnrades.

In einem Aspekt umfasst die Erfindung einen Kanüleninsertionsmechanismus für ein Patch Gerät, wobei das Zahnrad drehbar an der Basis angeordnet ist, so dass bei Freisetzung von Energie aus der Antriebsfeder, das Zahnrad relativ zum Schlitten in Rotation versetzbar ist.

In einem Aspekt umfasst die Erfindung einen Antrieb für einen Insertionsmechanismus für eine Verabreichungsvorrichtung aufweisend

- eine ebene oder gewölbte Basis
- eine in oder auf der Basis gebildete Führungsbahn, welche ein proximales und ein distales Ende und dazwischen eine Gerade oder eine Kurve definiert
- einen Schlitten rotationsfest an der Basis vorgesehen und entlang oder in der Führungsbahn zwischen einer proximalen Schlittenendposition und einer distalen Schlittenendposition verschiebbar, insbesondere hin und her bewegbar,
- ein Antriebsrad drehbar an der Basis gelagert,
- eine mit dem Schlitten verbundene Antriebsbahn aufweisend mehrere Antriebsabschnitte, wobei ein erster Antriebsabschnitt und ein zweiter Antriebsabschnitt sich gegenüberliegen und zur Gerade oder zur Kurve der Führungsbahn parallel verlaufen,
- eine Energiequelle, durch welche das Antriebsrad relativ zu Basis und Schlitten in Rotation versetzbar ist,

wobei bei Rotation des Antriebsrades der Schlitten ausgehend von insbesondere der proximalen Schlittenendposition durch einen form- oder kraftschlüssigen Eingriff des Antriebsrads in den ersten Antriebsabschnitt entlang oder in der Führungsbahn in distale Richtung verschiebt, bis am distalen Ende des ersten Antriebsabschnitts der form- oder kraftschlüssigen Eingriff des Antriebsrads vom ersten auf den zweiten Antriebsabschnitt durch ein Weiterdrehen des Antriebsrads wechselt und der Schlitten folglich entlang oder in der Führungsbahn in proximale Richtung verschiebt, wobei die Energiequelle über ein ein- oder mehrteiliges Übertragungselement und ein Einleitrad mit dem Antriebsrad koppelbar ist, wobei das Einleitrad verdrehgesichert mit dem Antriebsrad verbunden ist, und wobei das Übertragungselement verschiebbar oder rotierbar zur Basis angeordnet ist.

In einem Aspekt umfasst die Erfindung einen Antrieb für einen Insertionsmechanismus für eine Verabreichungsvorrichtung nach dem vorhergehenden Aspekt, wobei das Übertragungselement eine Zahnstange mit mindestens einer Zahnung ist und verschiebbar an der Basis angeordnet ist und wobei das Einleitrad ein Zahnrad ist, dessen Zähne in Eingriff mit einer Zahnung der Zahnstange bringbar sind.

In einem Aspekt umfasst die Erfindung einen Antrieb für einen Insertionsmechanismus für eine Verabreichungsvorrichtung nach dem vorhergehenden Aspekt, wobei die Basis weiter eine lineare Zahnstangenführung umfasst, insbesondere in Form einer Nut, und wobei die Zahnstange mindestens ein Führungselement umfasst, insbesondere mindestens zwei Nocken, über welches die Zahnstange mit der Basis verbunden ist und wobei die Zahnstange entlang der Zahnstangenführung verschiebbar ist.

In einem Aspekt umfasst die Erfindung einen Antrieb für einen Insertionsmechanismus für eine Verabreichungsvorrichtung nach dem vorhergehenden Aspekt, wobei die Energiequelle eine Zug- oder Druckfeder ist, wobei ein Ende der Zug- oder Druckfeder fest zur Basis angeordnet ist und zweites Ende fest an der Zahnstange angeordnet ist und wobei die Zug- oder Druckfeder für den Antrieb des Antriebs für den Insertionsmechanismus der Verabreichungsvorrichtung vorgespannt oder vorspannbar ist und wobei durch eine Freigabe der vorgespannten Zug- oder Druckfeder eine Kraft auf die Zahnstange wirkt, welche diese entlang der Zahnstangenführung in Bewegung versetzt und wodurch in der Folge Einleit- und Antriebsrad in eine Rotation versetzt werden.

In einem Aspekt umfasst die Erfindung einen Antrieb für einen Insertionsmechanismus für eine Verabreichungsvorrichtung einem vorhergehenden Aspekt, wobei der Antrieb weiter eine Freigabevorrichtung umfasst, wobei die Freigabevorrichtung mindestens folgende Elemente umfasst, ein fest an der Basis angeordnetes Halteelement, ein fest an der Zahnstange angeordnetes weiteres Halteelement, und ein Verbindungselement, mit welchem das Halteelement und das weitere Halteelement lösbar miteinander verbindbar sind, so dass, wenn das Halteelement und das weitere Halteelement über das Verbindungselement miteinander verbunden sind, die Zahnstange relativ zur Basis fest gehalten oder fixiert wird.

In einem Aspekt umfasst die Erfindung einen Antrieb für einen Insertionsmechanismus für eine Verabreichungsvorrichtung nach dem vorhergehenden Aspekt, wobei es sich beim Halteelement und dem weiteren Halteelement um Bohrungen handelt und das Verbindungselement als Stift oder Splint ausgebildet ist, welcher in die Bohrungen einführbar ist und dadurch die Zahnstange an der Basis fixierbar ist

In einem Aspekt umfasst die Erfindung einen Antrieb für einen Insertionsmechanismus für eine Verabreichungsvorrichtung, dabei weist die Zahnstange eine erste und eine zweite Zahnung auf, wobei die erste Zahnung in Eingriff mit dem als Zahnrad ausgebildeten Einleitrad bringbar ist und wobei die Energiequelle über die zweite Zahnung mit der Zahnstange kuppelbar ist.

In einem Aspekt umfasst die Erfindung einen Antrieb für einen Insertionsmechanismus für eine Verabreichungsvorrichtung, wobei die Energiequelle ein Elektromotor mit einer Elektromotorantriebsachse, welche durch den Elektromotor direkt oder über ein Getriebe in Rotation versetzbar ist, und wobei an der Elektromotorantriebsachse koaxial und fest ein Zahnrad angeordnet ist, welche in Eingriff mit der zweiten Zahnung der Zahnstange bringbar ist.

In einem Aspekt umfasst die Erfindung einen Antrieb für einen Insertionsmechanismus für eine Verabreichungsvorrichtung, wobei das Einleitrad als eine erstes Kegelrad, insbesondere als Kegelzahnrad, ausgeführt ist und das Übertragungselement ebenfalls als ein zweites Kegelrad, insbesondere als Kegelzahnrad ausgeführt ist, wobei erstes Kegelrad und zweites Kegelrad miteinander in Eingriff stehen, so dass eine Rotation des zweiten Kegelrades eine Rotation des ersten Kegelrades bewirkt und wobei die Rotationsachsen des ersten und des zweiten Kegelrades in einem Winkel von etwa 90° zueinander stehen.

In einem Aspekt umfasst die Erfindung einen Antrieb für einen Insertionsmechanismus für eine Verabreichungsvorrichtung, wobei das zweite Kegelrad durch einen elektrischen Antrieb oder einer Feder in Rotation versetzbar ist.

In einem Aspekt umfasst die Erfindung einen Antrieb für einen Insertionsmechanismus für eine Verabreichungsvorrichtung, wobei am Schlitten eine Zuleitung mit einem Lumen angeordnet und in oder am Schlitten geführt ist, durch welche eine zu verabreichende Substanz, insbesondere ein fluides Medikament, geleitet werden kann.

In einem Aspekt umfasst die Erfindung einen Antrieb für einen Insertionsmechanismus für eine Verabreichungsvorrichtung, wobei am distalen Ende des Schlittens eine Kanülenbrücke angeordnet ist, an welcher die Zuleitung endet und ein proximales Ende einer Insertionskanüle mit einem Lumen angeordnet ist, wobei das Lumen der Zuleitung und das Lumen der Insertionskanüle miteinander verbunden sind, so dass die zu verabreichende Substanz von der Zuleitung in die Insertionskanüle geleitet werden kann, und wobei die Insertionskanüle eine Verschiebung des Schlittens in distale wie in proximale Richtung mitmacht.

Ein Aspekt der Erfindung umfasst einen Kanüleninsertionsmechanismus für ein Patch Gerät, umfassend einen Antrieb wie vorhergehend beschrieben, eine Infusionskanüle mit einem distalen und einem proximalen Ende, durch welche die Insertionskanüle hindurchführbar ist, wobei am proximalen Ende der Infusionskanüle ein Kanülenträger fest angeordnet ist, wobei am Kanülenträger ein Verbindungmittel, insbesondere ein oder mehrere Schnapparme, angeordnet ist, über welches der Kanülenträger lösbar mit der Kanülenbrücke verbindbar ist, wenn die Insertionskanüle soweit durch die Infusionskanüle hindurch geführt ist, dass der Kanülenträger an der Kanülenbrücke anliegt.

Ein Aspekt der Erfindung umfasst ein Patch Gerät, insbesondere eine Patch Pumpe oder ein Patch Injektionsgerät, mit einem Kanüleninsertionsmechanismus nach dem vorhergehenden Aspekt.

In einem weiteren Aspekt umfasst die Erfindung einen Kanüleninsertionsmechanismus für ein Patch Gerät, wobei bei der Rotation des Zahnrades die Zähne des ersten Sektors des Zahnrades in einer ersten Phase in Eingriff mit der Zahnung der ersten Teilkulisse gelangen, wodurch über die Anordnung der ersten Teilkulisse am Schlitten der Schlitten entlang der Führungsbahn in Insertionsrichtung bewegt wird und wobei die Zähne des ersten Sektors des Zahnrades in einer zweiten Phase in Eingriff mit der Zahnung der zweiten Teilkulisse gelangen, wodurch über die Anordnungen der ersten Teilkulisse am Schlitten der Schlitten entlang der Führungsbahn entgegen der Insertionsrichtung bewegt wird.

In einem Aspekt umfasst die Erfindung einen Kanüleninsertionsmechanismus für ein Patch Gerät, wobei der erste Sektor des Zahnrades mit seinen Zähnen einen Teilkreisdurchmesser definiert, wodurch die sich aus dem Sektorwinkel des ersten Sektors und dem Teilkreisdurchmesser ergebende Bogenlinie den Insertionsweg der Kanüleninsertion ergeben.

**Figuren**

Figur 1     Explosionsdarstellung des der Erfindung zugrundeliegenden Insertionsmechanismus einer Patch Infusionspumpe 1

Figur 2     Ansicht der Teile der in Figur 1 gezeigten Patch Infusionspumpe in zusammengebautem Zustand (Ausgangszustand)

Figur 3a    Vertikaler Teilschnitt durch die Anordnung aus Figur 2

Figur 3b    Detail aus Figur 3a

Figur 4     Horizontalschnitt durch die Anordnung aus Figur 2 im Ausgangszustand

Figur 5     Ansicht des Insertionsmechanismus der Patch Infusionspumpe aus Figur 2 unmittelbar nach Freigabe des Insertionsmechanismus

Figur 6     Horizontalschnitt durch die Anordnung aus Figur 2 unmittelbar nach Freigabe des Insertionsmechanismus

Figur 7     Horizontalschnitt durch die Anordnung aus Figur 2 während der Insertionsbewegung

Figur 8     Horizontalschnitt durch die Anordnung aus Figur 2 nach erfolgter Insertion, vor dem Rückzug der Insertionskanüle 4a

Figur 9     Horizontalschnitt durch die Anordnung aus Figur 2 während des Rückzugs der Insertionskanüle 4a

Figur 10    Horizontalschnitt durch die Anordnung aus Figur 2 nach dem erfolgten Rückzug der Insertionskanüle 4a

| | |
|---|---|
| Figur 11 | Explosionsdarstellung eines erfindungsgemässen Kanüleninsertionsmechanismus für die Patch Infusionspumpe 100 |
| Figur 12 | Ansicht der Teile der in Figur 11 gezeigten Patch Infusionspumpe in zusammengebautem Zustand (Ausgangszustand) |
| Figur 13a | Aufsicht auf die Anordnung des Kanüleninsertionsmechanismus aus Figur 12 im Ausgangszustand |
| Figur 13b | Vertikalschnitt der Anordnung aus Figur 13a im Bereich des Auslösemechanismus |
| Figur 14a | Aufsicht auf die Anordnung des Kanüleninsertionsmechanismus aus Figur 12 unmittelbar nach Auslösung |
| Figur 14b | Vertikalschnitt der Anordnung aus Figur 14a im Bereich des Auslösemechanismus |
| Figur 15 | Aufsicht auf die Anordnung des Kanüleninsertionsmechanismus aus Figur 12 nach erfolgter Insertion, aber vor dem Rückzug der Insertionskanüle 104a |
| Figur 16 | Ansicht der Anordnung des Kanüleninsertionsmechanismus aus Figur 12 nach erfolgtem Rückzug der Insertionskanüle 104a (Endzustand) |
| Figur 17 | Aufsicht der Anordnung des Kanüleninsertionsmechanismus aus Figur 12 nach erfolgtem Rückzug der Insertionskanüle 104a |
| Figur 18 | Explosionsdarstellung eines erfindungsgemässen Kanüleninsertionsmechanismus für die Patch Infusionspumpe 200 |
| Figur 19 | Ansicht der Teile der in Figur 18 gezeigten Patch Infusionspumpe 200 in zusammengebautem Zustand (Ausgangszustand) |
| Figur 20 | Aufsicht auf die Anordnung des Kanüleninsertionsmechanismus aus Figur 19 nach erfolgter Insertion, aber vor dem Rückzug der Insertionskanüle 204a |
| Figur 21 | Aufsicht der Anordnung des Kanüleninsertionsmechanismus aus Figur 19 nach erfolgtem Rückzug der Insertionskanüle 204a (Endzustand) |
| Figur 22 | Explosionsdarstellung eines erfindungsgemässen Kanüleninsertionsmechanismus für die Patch Infusionspumpe 300 |
| Figur 23 | Ansicht der Teile der in Figur 22 gezeigten Patch Infusionspumpe 300 in zusammengebautem Zustand (Ausgangszustand) |
| Figur 24 | Ansicht auf die Anordnung des Kanüleninsertionsmechanismus aus Figur 23 nach erfolgter Insertion, aber vor dem Rückzug der Insertionskanüle 304a |
| Figur 25 | Ansicht der Anordnung des Kanüleninsertionsmechanismus aus Figur 23 nach erfolgtem Rückzug der Insertionskanüle 304a (Endzustand) |
| Figur 26 | Explosionsdarstellung eines erfindungsgemässen Kanüleninsertionsmechanismus für die Patch Infusionspumpe 400 |
| Figur 27 | Ansicht der Teile der in Figur 26 gezeigten Patch Infusionspumpe 400 in zusammengebautem Zustand (Ausgangszustand) |
| Figur 28 | Ansicht auf die Anordnung des Kanüleninsertionsmechanismus aus Figur 27 nach erfolgter Insertion, aber vor dem Rückzug der Insertionskanüle 404a |
| Figur 29 | Ansicht der Anordnung des Kanüleninsertionsmechanismus aus Figur 27 nach erfolgtem Rückzug der Insertionskanüle 404a (Endzustand) |

**Figurenbeschreibung**

Die folgenden Figurenbeschreibungen und Figuren zeigen und beschreiben verschiedene mögliche Ausführungen und Ausgestaltungen der Erfindung. Dabei handelt es sich nicht um eine abschliessende Aufzählung der möglichen Ausführungen der Erfindung, sondern lediglich um Beispiele. Dem Fachmann ergeben sich aus Beschreibung und Darstellung weitere Ausführungen der Erfindung, ohne dabei die Idee der Erfindung verlassen. Auch können durch Kombinationen von Teilen von beschriebenen Ausführungsformen und Gestaltungen weitere, dem Fachmann offensichtliche Ausführungsformen, Ausführungsformen entstehen, ohne dass dabei die Idee der Erfindung zu verlassen wird. Solche hier nicht gezeigten Ausführungsformen sind in dieser Schrift ausdrücklich mitgemeint.

Die folgenden Darstellungen und Ausführungsformen zeigen und beschreiben jeweils nicht ganze Verabreichungsgeräte, wie Patch Infusionspumpen oder Patch Injektionsgeräte, sondern der Übersichtlichkeit halber nur diejenigen Teile davon, welche für die Erfindung von Bedeutung sein könnten.

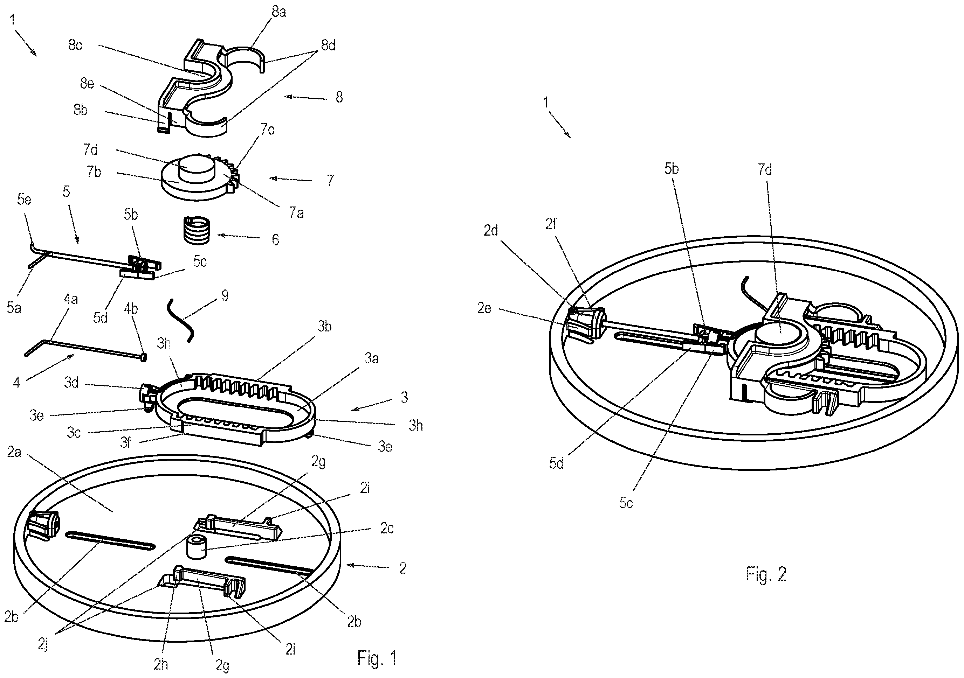

Die Figuren 1 bis 10 zeigen den grundlegenden Kanüleninsertionsmechanismus einer Patch Infusionspumpe 1, wie er die Basis für die erfindungsgemässen Ausführungsformen darstellt. Der in den Figuren 1 bis 10 dargestellte Kanüleninsertionsmechanismus ist bereits in der schweizerischen Patentanmeldung Nr 00062/17 ausführlich beschrieben, weshalb diese hier ausdrücklich vollständig durch Verweis in die vorliegende Anmeldung aufgenommen wird.

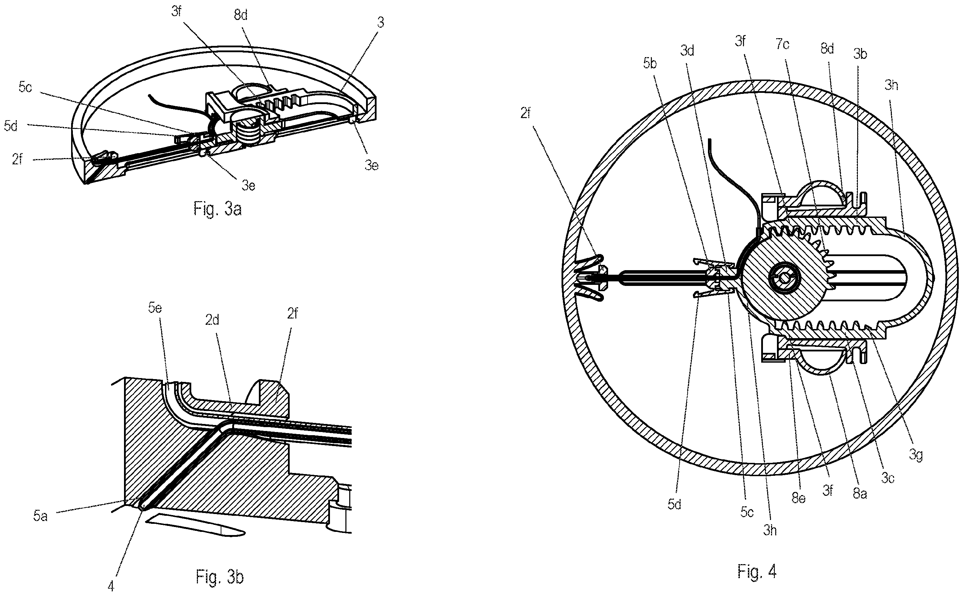

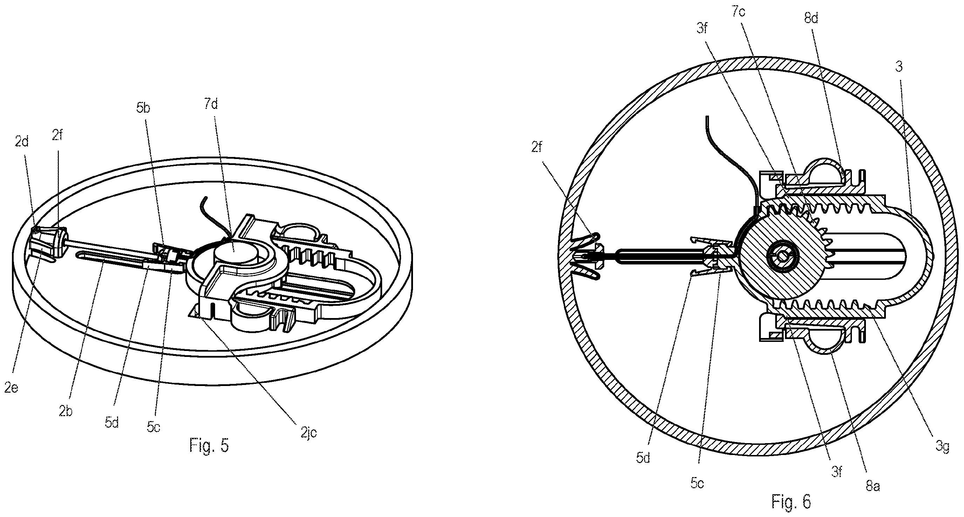

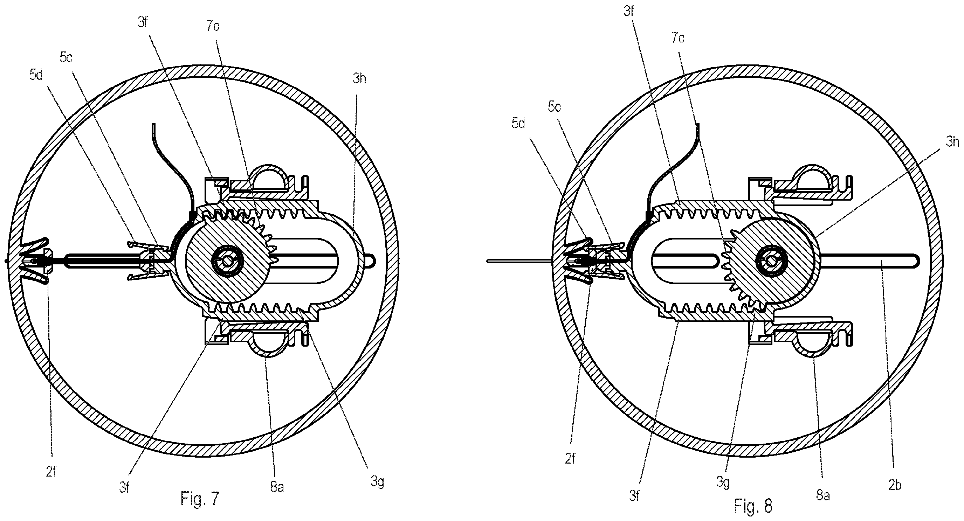

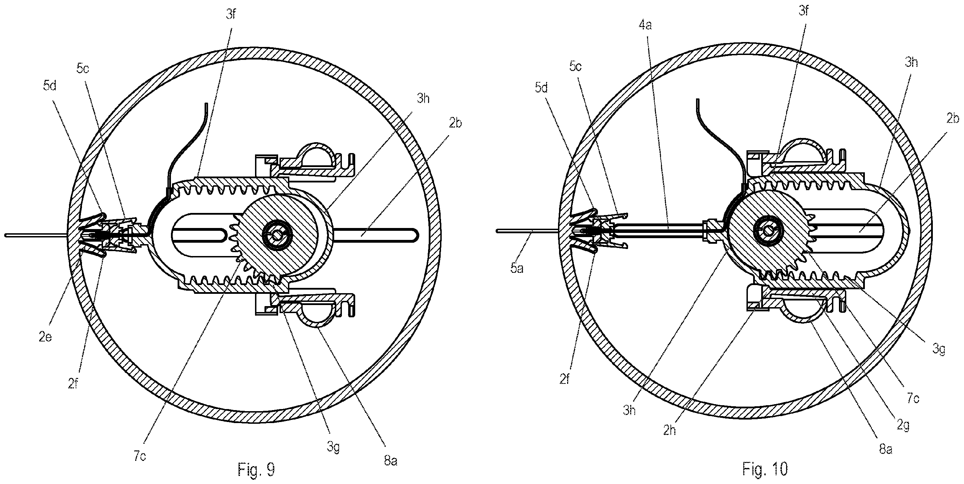

Figur 1 zeigt eine Explosionsdarstellung des grundlegenden Kanüleninsertionsmechanismus. Figur 2 zeigt eine Ansicht des grundlegenden Insertionsmechanismus im Ausgangszustand. Figur 3a zeigt einen Vertikalschnitt durch den grundlegenden Kanüleninsertionsmechanismus im Ausgangszustand. Figur 3b ein Detail aus Figur 3a. Figur 4 zeigt einen Horizontalschnitt durch den Kanüleninsertionsmechanismus im Ausgangszustand. Figuren 5 und 6 zeigen den Kanüleninsertionsmechanismus unmittelbar nach Auslösung des Mechanismus in zwei verschiedenen Ansichten. Figuren 7 bis 10 zeigen nun Horizontalschnitte durch den Kanüleninsertionsmechanismus in den weiteren Stadien des Insertionsprozesses, wobei Figur 10 den Endzustand darstellt.

Die Patch Infusionspumpe 1 umfasst ein Gehäuse 2, von welchem in den Figuren 1 bis 10 vor allem die Basis 2a zu sehen ist, welche ihrerseits als Gehäuseteil oder als Gehäuseeinsatz ausgestaltet sein kann. Das Gehäuse 2 wird im Bereich der Basis 2a mittels eines Pflasters auf die Haut der benutzenden Person aufgeklebt.

An der Basis 2a angeordnet sind die Führungsbahnen 2b, welche als Nuten aufgeführt sind. Weiter sind an der Basis die Federhalterung 2c, die Kanülenführung 2d, die Freigabearme 2g sowie die als Öffnungen in der Basis 2a ausgeführten Führungen 2j angeordnet. Der grundlegende Kanüleninsertionsmechanismus umfasst weiter einen Schlitten 3, welcher mit Hilfe der Führungselemente 3e verschiebbar in den Führungsbahnen 2b an der Basis geführt wird. Der Schlitten umfasst weiter die Kanülenbrücke 3d, an welcher die Insertionskanüle 4a mit ihrem Insertionskanülenträger 4b fest angeordnet ist (siehe hierzu speziell Figur 4). Die Insertionskanüle ist an ihren beiden Enden offen ausgestaltet, so dass Fluid durch die Insertionskanüle 4a hindurch geleitet werden kann. Ebenfalls fest mit der Kanülenbrücke 3d verbunden ist die Zuleitung 9, welche in Fluidverbindung mit der Insertionskanüle 4a steht und durch welche Fluid zum Beispiel aus einem Reservoir in die Insertionskanüle 4a geleitet werden kann. Die Zuleitung 9 verläuft wie in Figur 4 zu sehen teilweise am Schlitten 3. Der Schlitten 3, wie in den Figuren 1 bis 10 gezeigt, hat grob die Form eines Ovals. Das Oval hat die Funktion einer Führungskulisse 3a, welche aus der ersten Teilkulisse 3b, der zweiten Teilkulisse 3c sowie den beiden Bögen 3h besteht, welche die erste Teilkulisse 3b mit der zweiten Teilkulisse 3c verbinden. Das Innere des Ovals ist offen. Die erste Teilkulisse 3b und die zweite Teilkulisse 3c weisen einander gegenüberliegende Zahnungen auf.

An der Federhalterung 2c ist ein Ende der Antriebsfeder 6, im gezeigten Fall eine helixförmige Torsionsfeder, fest angeordnet, die Feder 6 ist dabei koaxial zur Federhalterung 2 angeordnet und wird von der Federhalterung auch gelagert. Das zweite Ende der Feder ist fest mit dem Zahnrad 7 verbunden, wobei das Zahnrad 7 drehbar zur der Federhalterung 2c auf derselben gelagert ist und wobei zwischen Federhalterung 2c und dem Zahnrad 7 die Feder 6 angeordnet ist. Die Zahnradachse 7d reicht durch das Oval des Schlittens 3 hindurch. Das Zahnrad 7 weist einen ersten Sektor 7a auf, welcher eine Zahnung mit Zähnen 7c aufweist. Weiter umfasst das Zahnrad 7 einen zweiten Sektor 7b ohne Zahnung. Die beiden Sektoren 7a und 7b werden vom erwähnten Oval gelagert, wobei die Zahnung des ersten Sektors 7a bei Rotation des Zahnrades 7 relativ zur Basis 2a und dem Schlitten 3 wechselweise in Eingriff mit den Zahnungen der erste Teilkulisse 3b und der zweiten Teilkulisse gelangen kann.

Der grundlegende Insertionsmechanismus umfasst ein Kanülenensemble 5 auf, welches einen Kanülenträger 5b umfasst. Am Kanülenträger 5b ist die Infusionskanüle 5a fest verankert. Im Ausgangzustand ragt die Insertionskanüle 4a durch den Kanülenträger 5b und das Lumen der Infusionskanüle 5a hindurch, wobei die distale Spitze der Insertionskanüle 4a aus dem distalen Ende der Infusionskanüle 5a herausragt. Wenn die Infusionskanüle 5a ins Gewebe der benutzenden Person eingeführt ist, wird die Insertionskanüle 3a durch die Infusionskanüle 5a hindurch zurückgezogen (aus dem Gewebe heraus), wobei die Insertionskanüle 4a nach abgeschlossenem Rückzug nach wie vor in die Infusionskanüle 5a hinein ragt und so die Fluidverbindung zwischen Zuleitung 9 und Infusionskanüle 5a herstellt, so dass die zu verabreichende Substanz der benutzenden Person zugeführt werden kann. Die Infusionskanüle 5a ist zusammen mit dem Kanülenträger 5b beweglich auf der Insertionskanüle 4a gelagert, wobei im Ausgangszustand Kanülenträger 5b und die Kanülenbrücke 3 über die Schnapparme 5c fest aber lösbar miteinander verbunden sind.

Die Figuren 3a und 3b zeigen teilweise Vertikalschnitte durch die Patch Infusionspumpe 1, wobei Figur 3b Details zur Kanülenführung 2d zeigt. Beim Insertionsvorgang bewegt sich der Schlitten 3 relativ zur Basis. Bei vielen Anwendungen ist die Bewegung des Schlittens und damit der Insertionskanüle 4a und auch der Infusionskanüle (beim Einstechen) in etwa parallel zur Hautoberfläche der benutzenden Person. Damit die Insertionskanüle 4a und die Infusionskanüle 5a im richtigen Winkel ins Gewebe geführt werden, leitet die Kanülenführung 2d die Insertionskanüle 4a und die Infusionskanüle 5a in Richtung Gewebe, was in Figur 3b gezeigt wird. In fakultativen Ausgestaltungen der Patch Infusionspumpe 1 umfasst das Kanülenensemble 5 einen Führungsschlauch 5e, dessen Aufgabe es ist, das Kanülenensemble 5 bei der Bewegung in die distale Richtung mechanisch zu stabilisieren (siehe Figur 3a und 3b). Der Führungsschlauch 5e umschliesst die Infusionskanüle 5a und ist mit einem Ende fest am Kanülenträger 5b angeordnet. Dabei weist der Führungsschlauch 5e auf seiner der Basis 2a zugerichteten Seite einen Längsschlitz auf. Wird das Kanülenensemble 5 in distale Richtung verschoben so wird der Führungsschlauch 5e entlang des Schlitzes im Bereich der Kanülenführung 2d geöffnet und von der Infusionskanüle 5a und der Insertionskanüle 4a abgetrennt und nicht durch die Basis 2a hindurch in Richtung Gewebe geleitet - im vorliegenden Beispiel von der Basis 2a weg.

Wie schon erwähnt ist das Zahnrad 7 rotierbar auf der Federhalterung 2c gelagert. Zwischen dem Zahnrad 7 und der Federhalterung 2c ist die Antriebsfeder 6 angeordnet, welche als helikale Torsionsfeder ausgeführt ist. Es sei an dieser Stelle noch einmal erwähnt, dass die Antriebsfeder auch andere Formen haben könnte, insbesondere könnte zum Beispiel die Feder mit der Federhalterung integriert als Torsionsstab ausgestaltet sein, welcher an einem Ende mit dem Zahnrad 7 fest verbunden wäre. Zurück zur gezeigten Ausführung: ein Ende der Antriebsfeder 6 ist fest mit der Federhalterung 2c verbunden, das andere fest mit dem Zahnrad 7. Energie, welche in der Feder 6 gespeichert ist, kann so dann in eine Rotation des Zahnrades 7 relativ zur Basis 2a umgesetzt werden. Wie erwähnt ist das Zahnrad 7 in einen ersten Sektor 7a mit Zähnen 7c sowie einen zweiten Sektor 7b ohne Zähne aufgeteilt. Die Führungskulisse 3a ist wie erwähnt fest am Schlitten 3 angeordnet und umfasst di erste Teilkulisse 3b und eine zweite Teilkulisse 3c.

Im Ausgangszustand ist die Feder 6 vorgespannt, so dass ein Moment auf das Zahnrad 7 wirkt, welches über die Zähne 7c auf die erste Teilkulisse 3b am Schlitten 3 übertragen wird. In der Folge drückt der Schlitten 3 in distale Richtung. Im Ausgangszustand wird diese Bewegung jedoch blockiert, wie zum Beispiel in Figur 4 zu sehen. Dazu sind an der Basis Freigabearme 2g angeordnet, welche ein freies Ende mit Zahn 2h aufweisen. Jeweils ein Zahn 2h ist im Ausgangszustand in Eingriff mit einem Zahn 3f des Schlittens 3 (der grundlegende Insertionsmechanismus weist in der gezeigten Variante, jeweils einen solchen Zahn 3f auf beiden Seiten des Schlittens 3 auf). Um eine Flexion des freien Endes 2h des Freigabearmes 2g nach aussen im Ausgangszustand zu verhindern, umfasst der Insertionsmechanismus der Patch Infusionspumpe 1 weiter eine Freigabeklammer 8.

Die Freigabeklammer 8 ist über die Führungsarme 8b, welche in den Führungen 2j verschiebbar gelagert sind, mit der Basis 2a verbunden. Die Freigabeklammer umfasst weiter die Federelemente 8a, deren freie Enden 8d an den Ausbuchtungen 2i der Freigabearme 2g anstehen und so federnd die Freigabeklammer in distale Richtung drücken. Die Bucht 8c der Freigabeklammer 8 lagert dabei die Zahnradachse 7d und drückt das Zahnrad ebenfalls in distale Richtung (siehe zum Beispiel Figur 2).

Wie erwähnt zeigt Figur 4 einen Horizontalschnitt durch den Insertionsmechanismus im Ausgangszustand. Hier wird klar, wie die Blockierzonen 8e der Freigabeklammer 8 ein Auslenken der freien Enden der Freigabearme 2g verhindern. Der Insertionsmechanismus kann nun durch ein Verschieben der Freigabeklammer in proximale Richtung entgegen der durch die Federelemente 8a induzierte Federkraft freigegeben werden. Durch die Verschiebung der Freigabeklammer 8 in proximale Richtung verschieben sich auch die Blockierzonen 8e in proximale Richtung.

In den Figuren 5 und 6 ist der Insertionsmechanismus bei betätigter Freigabeklammer 8 gezeigt. In diesem Zustand können nun die freien Enden 2h der Freigabearme 2g nach aussen flektieren, wodurch eine Bewegung des Schlittens 3 in distale Richtung entlang der Führungsbahnen 2b möglich wird. Das auf das Zahnrad 7 wirkende Drehmoment aus der Feder 6 bewirkt nun, dass das Zahnrad zu rotieren beginnt, und zwar im gezeigten grundlegenden Insertionsmechanismus der Patch Infusionspumpe 1 im Gegenuhrzeigersinn. Entsprechend verschiebt sich der Schlitten 3 in distale Richtung, wodurch das Kanülenensemble 5 und das Insertionskanülenensemble 4 ebenfalls in Insertionsrichtung verschoben werden, was zum Einführen von Insertionskanüle 4a und Infusionskanüle 5a ins Gewebe der benutzenden Person führt. Figur 7 zeigt den Insertionsmechanismus während der Bewegung in Insertionsrichtung.

Die Bewegung in distale Richtung geht weiter bis die Zähne 7c aus der Zahnung der ersten Teilkulisse 3b heraus ausser Eingriff gelangen. Gleichzeitig erreicht der Kanülenträger 5b die Kanülenführung 2d. Kanülenführung 2d umfasst eine trichterartig zulaufende Führung 2e, welche so dann die Haltearme 5d des Kanülenträgers 5b nach innen auslenkt, wodurch diese mit dem Kanülenhalter 2f in Eingriff gebracht werden. Durch die Auslenkung der Haltearme 5d nach innen, werden umgekehrt die Schnapparme 5c nach aussen ausgelenkt, wodurch der Eingriff der Schnapparme 5c in die Kanülenbrücke 3d gelöst wird. Da immer noch ein Drehmoment am Zahnrad 7 anliegt, dreht sich dieses während dessen weiter in Gegenuhrzeigersinn (Figur 8).

In Figur 8 ist der Insertionsmechanismus bei erfolgter Insertion der Kanülen 4a und 5a gezeigt, aber noch vor dem Rückzug der Insertionskanüle 4a. Das Zahnrad 7 rotiert zwischen der Insertion und vor dem Rückzug der Insertionskanüle 4a über einen Winkel, bei welchem die Zähne 7c weder in Eingriff mit der ersten Teilkulisse 3b noch der zweiten Teilkulisse 3c sind. Hierbei bleibt der Schlitten stehen. Figur 8 zeigt den Moment, in welchem die Zähne 7c gerade in Eingriff mit der zweiten Teilkulisse 3c gelangen. Zu beachten ist, dass aufgrund der Dimensionierung des ersten Sektors 7a und des Kreisdurchmessers des Zahnrades 7 der proximalste Zahn der zweiten Teilkulisse, Halbzahn 3g, abschnitten ist, damit die Bewegung des Zahnrades 7 nicht durch ungewünschte Interferenz mit der zweiten Teilkulisse 3c blockiert wird. Weiter zu beachten ist, dass im Zustand von Figur 8 der Kanülenträger 5b mit dem Kanülenhalter 2f fest verbunden ist und damit die Infusionskanüle 5a beim Rückzug der Insertionskanüle 4a an Ort verbleibt.

In Figur 9 ist die Rückzugsbewegung des Schlittens bereits in Gang, das heisst, der Schlitten 3 bewegt sich entgegen der Insertionsrichtung. Der Schlitten 3 hat die Bewegungsrichtung geändert, wobei das Zahnrad 7 immer in dieselbe Richtung rotiert.

Das Ende des Rückzugs ist in Figur 10 dargestellt. In diesem Zustand verhindert der distale Bogen 3h des distalen Endes der Führungskulisse 3a eine weitere Bewegung des Schlittens 3 in proximale Richtung, in dem der Bogen 3h in Anschlag mit der Zahnradachse 7d des Zahnrads 7 gelangt. Durch die Tatsache, dass ein Teil der Zähne 7c weiter in Eingriff mit der Zahnung der zweiten Teilkulisse 3c ist, ist auch eine weitere Rotation des Zahnrades 7 nicht mehr möglich. Der Insertionsmechanismus hat in der Folge seinen Endzustand erreicht.

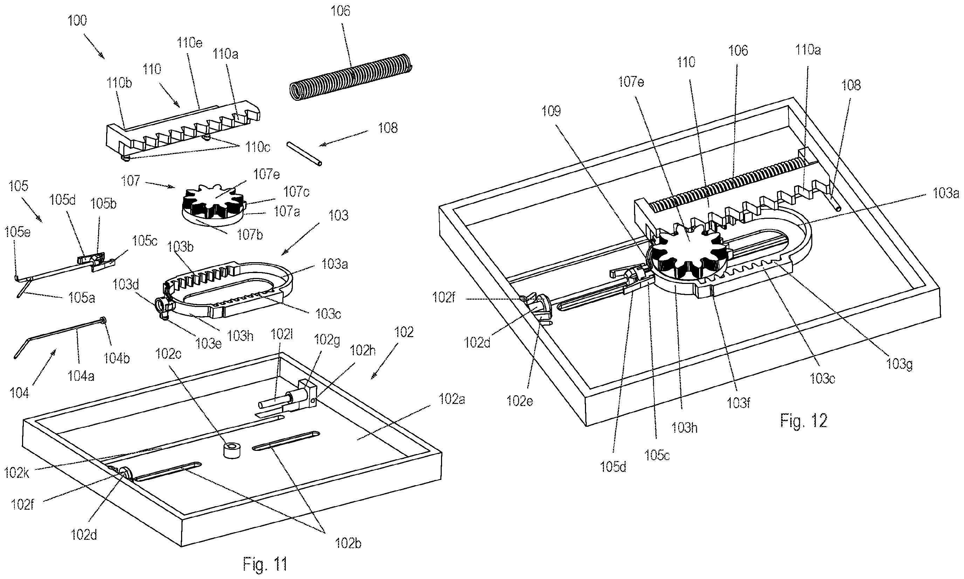

Eine erfindungsgemässe Ausführungsform des Kanüleninsertionsmechanismus ist in den Figuren 11 bis 17 dargestellt. Die Bezeichner bei dieser Ausführungsform wurden analog zum oben dargestellten grundlegenden Kanüleninsertionsmechanismus von Figur 1 benannt, die Nummerierung wurde so vorgenommen, dass z. B. die Basis 2a der Gestaltung aus Figur 1 analog dem Teil 102a der Ausführungsform aus Figur 11 ist. Da auch die Funktion der Ausführungsform aus Figur 11 sehr ähnlich zur Funktion des Kanüleninsertionsmechanismus aus Figur 1 ist, wird im Folgenden insbesondere auf die Unterschiede zwischen den Mechanismen eingegangen.

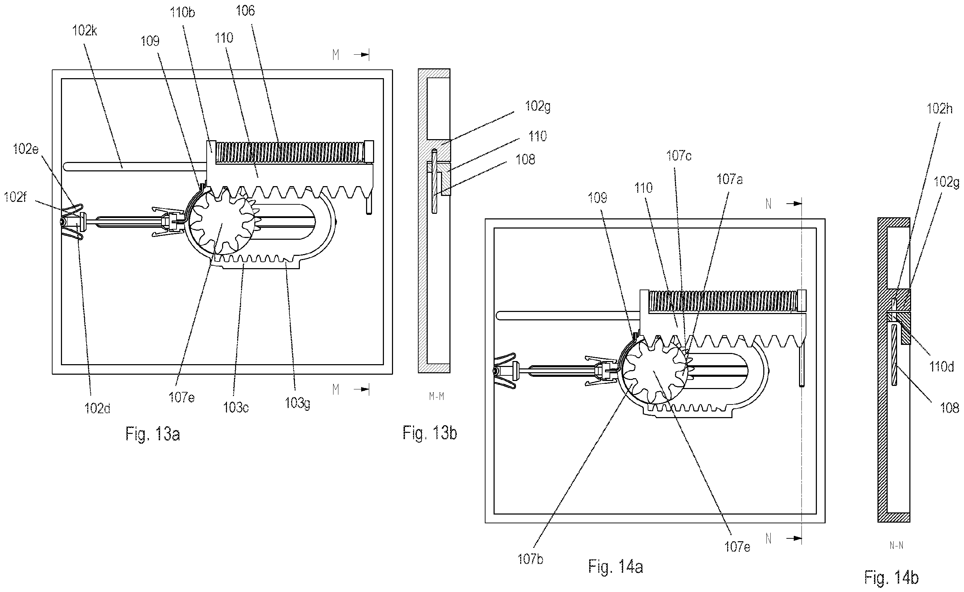

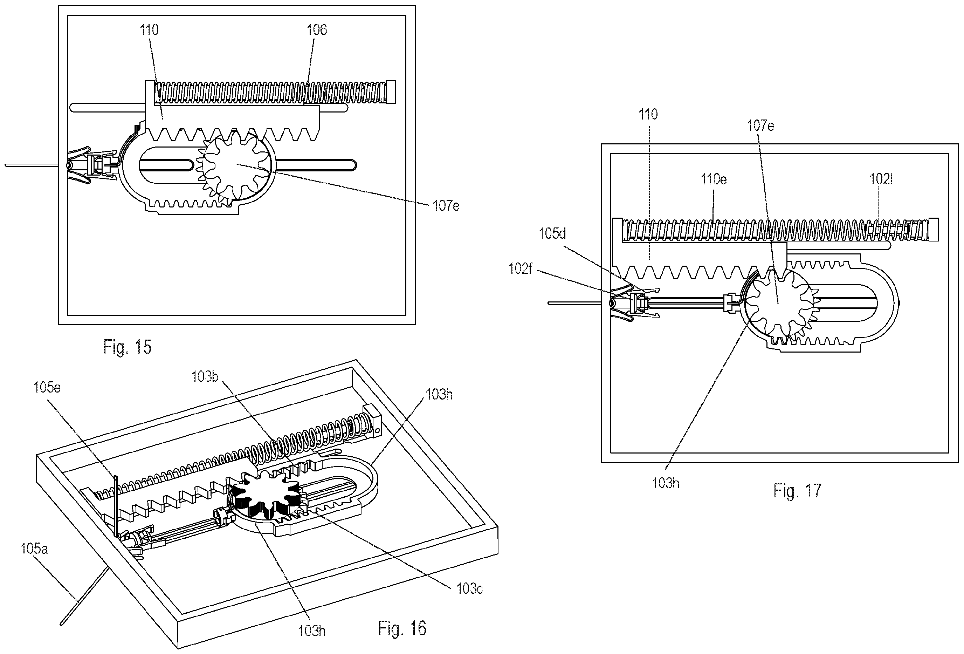

Figur 11 zeigt eine Explosionsdarstellung von Teilen des Patch Infusionsgerätes 100. Figur 12 zeigt eine Ansicht der Teile nach dem Zusammenbau im Ausgangszustand. Figur 13a zeigt eine Aufsicht auf die Anordnung aus Figur 12 im Ausgangszustand während Figur 14a die Anordnung unmittelbar nach dem Auslösen des Kanüleninsertionsmechanismus durch das Entfernen des Auslösestiftes 108 zeigt. Die Figuren 13b und 14b zeigen die Zustände aus den Figuren 13a respektive 14a in den Vertikalschnitten M-M und N-N. Figur 15 zeigt die Anordnung aus Figur 12 nach erfolgter Insertion der Infusionskanüle 105a, aber vor dem Rückzug der Insertionskanüle 104a. Die Figuren 16 respektive 17 zeigen die Anordnung aus Figur 12 im Endzustand des Kanüleninsertionsmechanismus.

Wie erwähnt zeigen die Darstellungen in den Figuren jeweils nicht die ganze Patch Infusionspumpe 100, sondern der Übersichtlichkeit halber nur diejenigen Teile davon, welche für die Erfindung von Bedeutung sein könnten.

Ein Unterschied zwischen Patch Infusionspumpe 1 und Patch Infusionspumpe 100 liegt im Antrieb des Kanüleninsertionsmechanismus. Das Zahnrad 107 wird nicht direkt durch eine Torsionsfeder 6 angetrieben. Die Antriebsenergie für den Kanüleninsertionsmechanismus der Patch Infusionspumpe 100 stammt aus der Antriebsfeder 106, welche als Druckfeder ausgebildet ist. Ein Ende der Druckfeder 106 ist am Druckfederwiderlager 102g angeordnet, wobei das Druckfederwiderlager fest an der Basis 102a angeordnet ist, resp. alternativ einteilig mit der Basis ausgestaltet sein kann. Das Druckfederwiderlager 102g weist eine stiftförmige Verlängerung 102l auf, welcher der Feder 106 als Führung dienen kann. Das zweite Ende der Druckfeder 106 ist an der Zahnstange 110, genauer am Federlager 110b und dessen stiftartigen Verlängerung 110e gelagert. Die Zahnstange 110 ist verschiebbar, insbesondere linear verschiebbar, an der Basis 102a gelagert, dazu umfasst die Zahnstange die insbesondere nockenförmigen Führungselemente 110c (siehe hierzu Figur 11) und die Basis 102a umfasst die insbesondere nutenförmige Zahnstangenführung 102k, in welcher die Führungselemente 110c geführt werden. Figur 12 zeigt den Kanüleninsertionsmechanismus im Ausgangszustand, in welchem die Druckfeder 106 gespannt (komprimiert) ist. Figur 17 zeigt den Kanüleninsertionsmechanismus nach der Insertion der Infusionskanüle 105a und dem Rückzug der Insertionskanüle 104a in seinem Endzustand bei zumindest teilweise entspannter Druckfeder 106. Während der Entspannungsbewegung der Feder 106 hat diese die Zahnstange 110 in distale Richtung verschoben.

Zahnstange 110 umfasst die Zahnung 110a, welche in Eingriff mit der Zahnung des Einleitrades 107e steht, so dass eine Verschiebungsbewegung der Zahnstange 110 in eine Rotation des Einleitrades 107e umgewandelt werden kann. Das Einleitrad 107e ist, wie in den Figuren gezeigt, als Zahnrad ausgestaltet. In der vorliegenden Ausführungsform ist das Einleitrad 107e fest mit dem Zahnrad 107 verbunden, oder alternativ einteilig mit dem Zahnrad 107 ausgeführt. Einleitrad 107e und Zahnrad 107 sind zu einander koaxial angeordnet, wobei das Zahnrad 107 drehbar am Zahnradlager 102c angeordnet ist. In der gezeigten Ausführung liegen das Einleitrad 107e und das Zahnrad 107 direkt auf einander. In alternativen Ausführungen könnte es zwischen dem Einleitrad 107e und dem Zahnrad 107 auch einen Abstandgeben und die beiden Teile wären zum Beispiel über eine gemeinsame Achse fest miteinander verbunden. Zurück zur gezeigten Ausführungsform: wird also die Zahnstange 110 entlang der Zahnstangenführung 102k verschoben, so bewirkt die Verschiebung über den Eingriff von Zahnung 110a und Einleitrad 107e eine Rotation Einleitrades 107e und somit eine Rotation des Zahnrades 107 relativ zur Basis. Die Einleitung der Antriebsenergie für den Kanüleninsertionsmechanismus erfolgt also von einer Antriebsfeder auf ein Übertragungselement, in diesem Fall die Zahnstange 110, und vom Übertragungselement auf das Einleitrad 107e, welches seinerseits seine Bewegung auf das Zahnrad 107 überträgt.

Ein weiterer Unterschied zwischen den Kanüleninsertionsmechanismen der Patch Infusionspumpe 1 und der Patch Infusionspumpe 100 liegt in der Freigabe der Kanüleninsertionsmechanismen. Die Freigabe für den Kanüleninsertionsmechanismus der Patch Infusionspumpe 100 ist deutlich einfacher aufgebaut als diejenige der Patch Infusionspumpe 1. Im Folgenden wird insbesondere Bezug genommen auf die Figuren 13a bis 14b. Im Ausgangszustand verbindet der Freigabestift 108 das Druckfederwiderlager 102g und die Zahnstange 110, indem der Freigabestift durch die Bohrung 110d der Zahnstange hindurch in die Bohrung 102h des Druckfederwiderlagers 102g geführt ist, wodurch eine Expansion der Druckfeder 106 blockiert ist (siehe hierzu die Figuren 13a und 13b). Zusätzlich wird durch den bestehenden Eingriff von Zahnstange 110 und Einleitrad 107e auch eine Rotation des Zahnrades 107 und folglich eine Verschiebung des Schlittens 103 unterbunden. Der Mechanismus wird freigegeben, indem der Freigabestift aus den Bohrungen heraus gezogen wird, wie ein Splint, wodurch die Zahnstange 110 entlang der Zahnstangenführung 102k verschiebbar wird. Die sich entspannende Druckfeder bewirkt diese Verschiebung, welche ihrerseits einen Antrieb des Kanüleninsertionsmechanismus zur Folge hat. In den Figuren ist der Stift 108 geometrisch sehr einfach ausgestaltet. Alternativ könnte der Stift jedoch als Splint ausgeführt sein, welcher durch die Gehäusewandung 102 aus der Patch Infusionspumpe 100 heraus geführt wird, so dass eine einfache manuelle Auslösung des Kanüleninsertionsmechanismus durch die benutzende Person ermöglicht wird.

Die Freigabe des Kanüleninsertionsmechanismus könnte auch gleich wie beim grundlegenden Kanüleninsertionsmechanismus von Patch Infusionspumpe 1 ausgestaltet sein, ohne von der Erfindungsidee abzuweichen.

Für die weitere Funktionsbeschreibung des Kanüleninsertionsmechanismus der Patch Infusionspumpe 100 wird auf die Beschreibung des Kanüleninsertionsmechanismus der Patch Infusionspumpe 1 verwiesen, welcher mit Ausnahme der beschriebenen Unterschiede analog oder gleich funktioniert. Hier sei noch einmal angemerkt, dass analoge Teile analoge Bezeichner tragen, so entspricht der Schlitten 3 der Patch Infusionspumpe 1 dem Schlitten 103 der Patch Infusionspumpe 100.

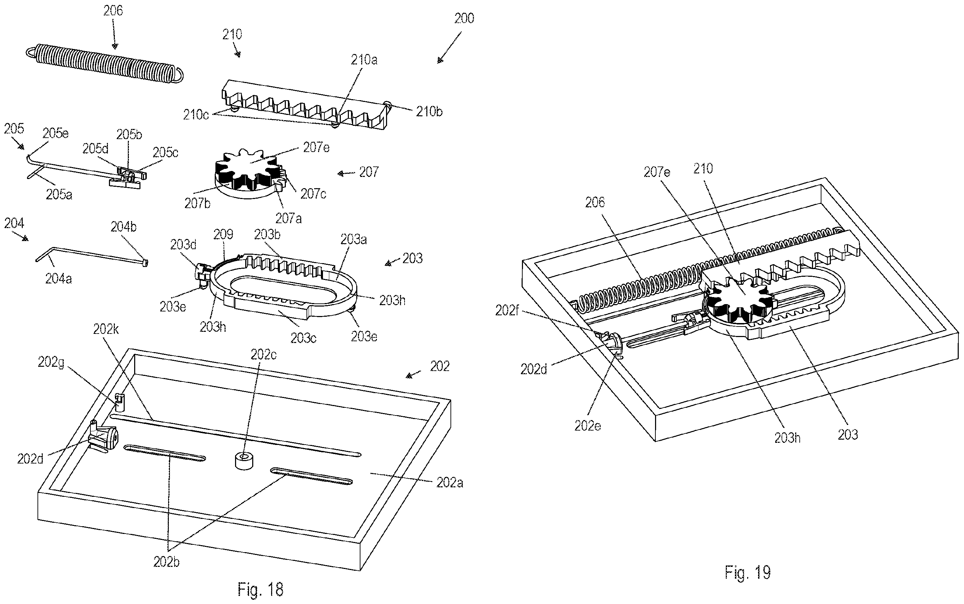

Eine erfindungsgemässe Ausführungsform des Kanüleninsertionsmechanismus ist in den Figuren 18 bis 21 dargestellt. Die Bezeichner bei dieser Ausführungsform wurden analog zum oben dargestellten grundlegenden Kanüleninsertionsmechanismus von Figur 1 benannt, die Nummerierung wurde so vorgenommen, dass z. B. die Basis 2a der Gestaltung aus Figur 1 analog dem Teil 202a der Ausführungsform aus Figur 18 ist. Da die Funktion der Ausführungsform aus Figur 18 sehr ähnlich zur Funktion des Kanüleninsertionsmechanismus aus Figur 11 ist, wird im Folgenden insbesondere auf die Unterschiede zwischen den Mechanismen eingegangen. Der Übersicht halber wird bei der Ausführungsform aus den Figuren 18 bis 21 kein Freigabemechanismus gezeigt. Es ist jedoch so, dass entweder der Freigabemechanismus aus dem grundlegenden Insertionsmechanismus (Figuren 1 bis 10) oder auch derjenige aus der vorhergehenden erfindungsgemässen Ausführungsform eingesetzt werden kann. Die Mittel zur Umsetzung des Freigabemechanismus sind dem Fachmann basierend auf der vorliegenden Offenbarung naheliegend.

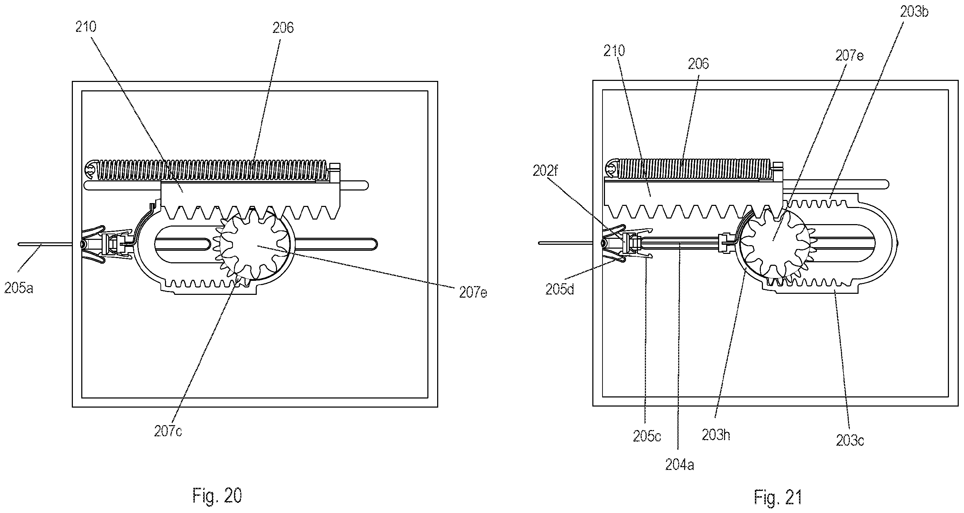

Figur 18 zeigt eine Explosionsdarstellung von Teilen des Patch Infusionsgerätes 200. Figur 19 zeigt eine Ansicht der Teile nach dem Zusammenbau im Ausgangszustand. Figur 20 zeigt die Anordnung aus Figur 19 nach erfolgter Insertion der Infusionskanüle 205a, aber vor dem Rückzug der Insertionskanüle 204a. Die Figur 21 zeigt die Anordnung aus Figur 19 im Endzustand des Kanüleninsertionsmechanismus.

Die erfindungsgemässe Ausführungsform der Figuren 18 bis 21 zeigt eine zur vorhergehenden Ausführungsform unterschiedliche Energiequelle. Statt der Druckfeder 106 gelangt in der Ausführungsform der Figuren 18-21 eine Zugfeder 206 zum Einsatz, um den Kanüleninsertionsmechanismus anzutreiben. Die Zugfeder 206 ist mit einem Ende am Federlager 202g der Basis 202a verankert und mit dem anderen Ende am Federlager 210b der Zahnstange 210. Bis auf diesen Unterschied im Antrieb funktioniert die Ausführungsform aus den Figuren 18 bis 21 gleich wie die Ausführungsform aus den Figuren 11 bis 17.

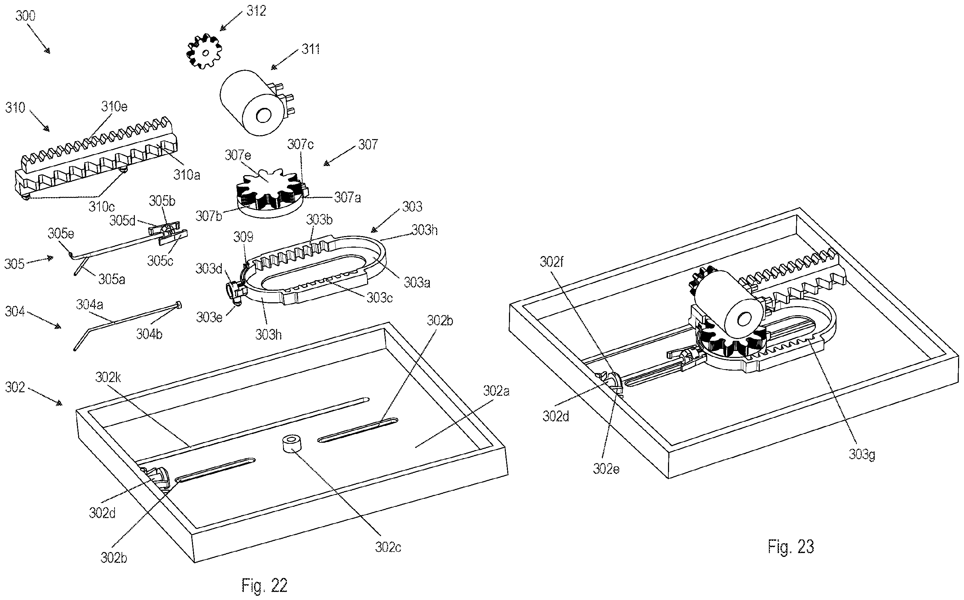

Eine erfindungsgemässe Ausführungsform des Kanüleninsertionsmechanismus ist in den Figuren 22 bis 25 dargestellt. Die Bezeichner bei dieser Ausführungsform wurden analog zum oben dargestellten grundlegenden Kanüleninsertionsmechanismus von Figur 1 benannt, die Nummerierung wurde so vorgenommen, dass z. B. die Basis 2a der Gestaltung aus Figur 1 analog dem Teil 302a der Ausführungsform aus Figur 22 ist. Da die Funktion der Ausführungsform aus Figur 22 sehr ähnlich zur Funktion des Kanüleninsertionsmechanismus aus Figur 11 ist, wird im Folgenden insbesondere auf die Unterschiede zwischen den Mechanismen eingegangen. Der Übersicht halber wird bei der Ausführungsform aus den Figuren 22 bis 25 ebenfalls kein Freigabemechanismus gezeigt. Es ist jedoch so, dass entweder der Freigabemechanismus aus dem grundlegenden Insertionsmechanismus (Figuren 1 bis 10) oder auch derjenige aus der erfindungsgemässen Ausführungsform aus den Figuren 11 bis 17 eingesetzt werden könnte. Die Mittel zur Umsetzung des Freigabemechanismus sind dem Fachmann basierend auf der vorliegenden Offenbarung naheliegend.

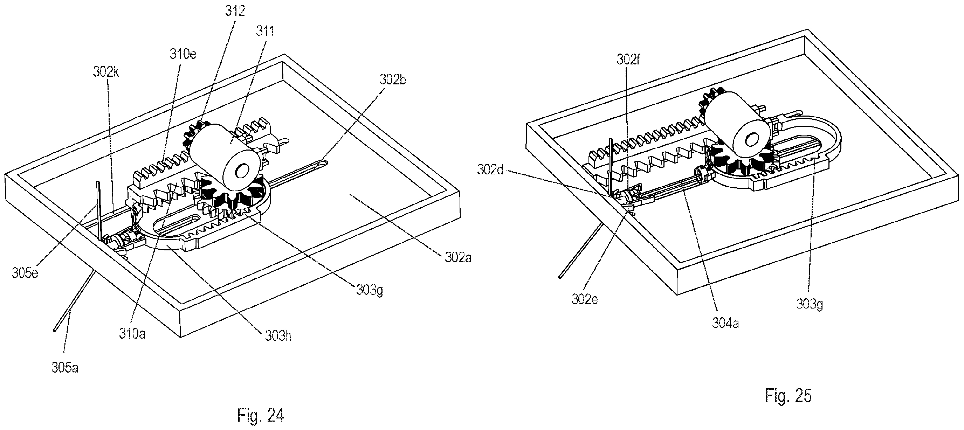

Figur 22 zeigt eine Explosionsdarstellung von Teilen des Patch Infusionsgerätes 300. Figur 23 zeigt eine Ansicht der Teile nach dem Zusammenbau im Ausgangszustand. Figur 24 zeigt die Anordnung aus Figur 23 nach erfolgter Insertion der Infusionskanüle 305a, aber vor dem Rückzug der Insertionskanüle 304a. Die Figur 25 zeigt die Anordnung aus Figur 23 im Endzustand des Kanüleninsertionsmechanismus.

Bei dieser erfindungsgemässen Ausführungsform wird die Energiequelle für den Kanüleninsertionsmechanismus nicht durch eine Feder gebildet, sondern durch den Elektromotor 311, welcher das Zahnrad 312 in Rotation versetzt, mit welchem die Rotationsachse des Elektromotors 311 fest verbunden ist. Der Motor ist mit geeigneten Mittel am Gehäuse 302 oder der Basis 302a befestigt (nicht gezeigt in den Figuren). Das Zahnrad 312 greift in die zweite Zahnung 310e ein, welche fest an der Zahnstange 310 angeordnet ist. Die Zahnstange 310 hat die gleiche Funktion wie schon die Zahnstangen 110 und 210 der vorhergehend beschriebenen Ausführungsformen. Eine Rotation des Zahnrades 312 wird über die zweite Zahnung 310e der Zahnstange in eine Verschiebung der Zahnstange 310 entlang der Zahnstangenführung 302k umgesetzt, wobei die Verschiebung der Zahnstange 310 über die Zahnung 310a in eine Rotation des Einleitrades 307e umgewandelt wird. Wie schon in den vorhergehenden Ausführungsformen ist das Einleitrad 307e fest mit dem Zahnrad 307 verbunden, welches durch die Rotation des Einleitrades ebenfalls rotiert. Wie in der Beschreibung des grundlegenden Kanüleninsertionsmechanismus bewirkt die Rotation des Zahnrades 307 zuerst eine Verschiebung des Schlittens 303 in distale Richtung (Insertion der Infusionskanüle 305a) und folgend eine Verschiebung des Schlittens in proximale Richtung (Rückzug der Insertionskanüle 304a). Bis auf den unterschiedlichen Antrieb funktioniert der Kanüleninsertionsmechanismus gleich wie die vorgängig beschriebenen Kanüleninsertionsmechanismen der Figuren 1 bis 21.

Ein Vorteil der Ausführungsform der Figuren 22 bis 25 ergibt sich aus der Tatsache, dass ein Elektromotor als Antrieb verwendet wird. Der Elektromotor 311 kann so ausgelegt sein, die Rotationsachse des Motors 311 im Ruhezustand nur gegen ein Rastmoment rotierbar ist, also ein erheblicher Widerstand gegen Rotation besteht. Dazu kann der Motor 311 zum Beispiel als DC Motor mit Permanentmagnet ausgebildet sein. Alternativ kann der Motor 311 als Schrittmotor ausgebildet sein und zum Beispiel aktiv in einer Position gehalten werden. Dieser Widerstand gegen Rotation im Ruhezustand kann den Vorteil haben, dass kein eigentlicher Freigabemechanismus, wie oben beschrieben, vorhanden sein muss, und der Kanüleninsertionsmechanismus über eine Aktivierung des Elektromotors 311 betätigt werden kann.

Ein weiterer Vorteil des Einsatzes eines Elektromotors kann darin gesehen werden, dass das Beenden der Rückzugsbewegung des Schlittens 303 durch Stoppen des Motors erfolgen kann, das heisst dass der Rückzug der Insertionskanüle gestoppt werden kann, bevor das Zahnrad 307 in Anschlag mit einem der Bogen 303h gelangt. Bei dieser Ausführungsform könnte also der Bogen 303h grundsätzlich weggelassen werden und Start und Stopp des Bewegungsablaufs des Kanüleninsertionsmechanismus wie auch dessen Anfangs- und Endposition durch die Steuerung des Motors gegeben werden.

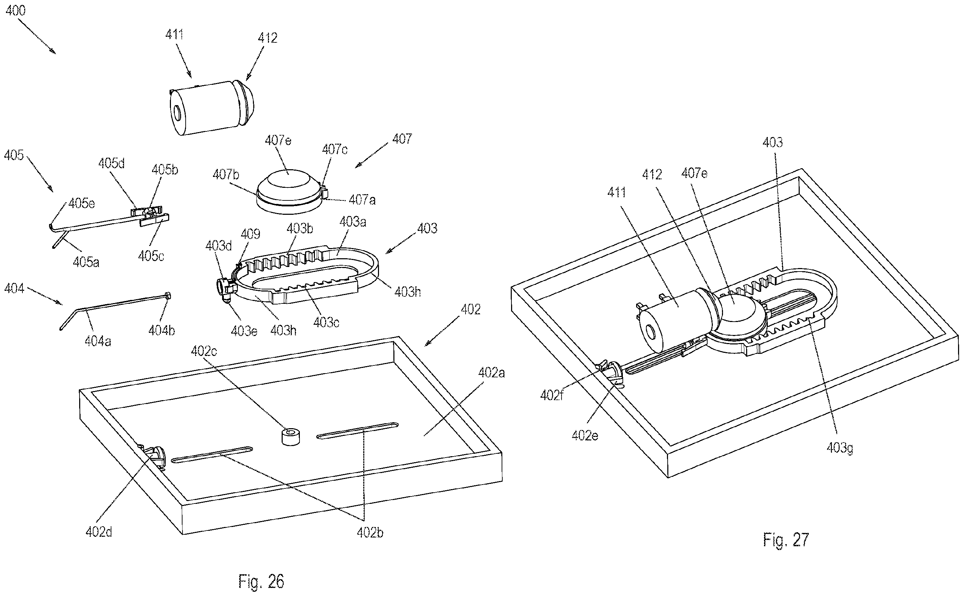

Eine erfindungsgemässe Ausführungsform des Kanüleninsertionsmechanismus ist in den Figuren 26 bis 29 dargestellt. Die Bezeichner bei dieser Ausführungsform wurden analog zum oben dargestellten grundlegenden Kanüleninsertionsmechanismus von Figur 1 benannt, die Nummerierung wurde so vorgenommen, dass z. B. die Basis 2a der Gestaltung aus Figur 1 analog dem Teil 402a der Ausführungsform aus Figur 22 ist. Da die Funktion der Ausführungsform aus Figur 2 sehr ähnlich zur Funktion des Kanüleninsertionsmechanismus aus Figur 22 ist, wird im Folgenden insbesondere auf die Unterschiede zwischen den Mechanismen eingegangen. Der Übersicht halber wird bei der Ausführungsform aus den Figuren 26 bis 29 ebenfalls kein Freigabemechanismus gezeigt. Es ist jedoch so, dass entweder der Freigabemechanismus aus dem grundlegenden Insertionsmechanismus (Figuren 1 bis 10) oder auch derjenige aus der erfindungsgemässen Ausführungsform aus den Figuren 11 bis 17 eingesetzt werden könnte. Die Mittel zur Umsetzung des Freigabemechanismus sind dem Fachmann basierend auf der vorliegenden Offenbarung naheliegend.

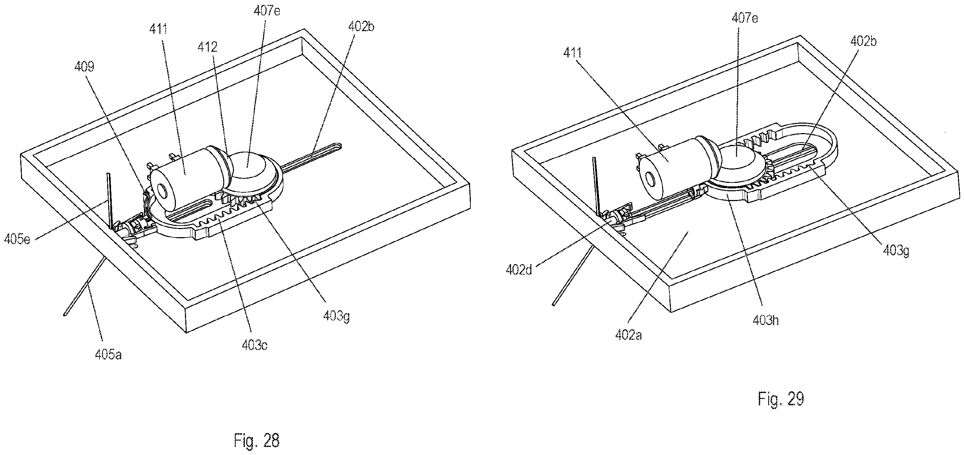

Figur 26 zeigt eine Explosionsdarstellung von Teilen des Patch Infusionsgerätes 400. Figur 27 zeigt eine Ansicht der Teile nach dem Zusammenbau im Ausgangszustand. Figur 28 zeigt die Anordnung aus Figur 27 nach erfolgter Insertion der Infusionskanüle 405a, aber vor dem Rückzug der Insertionskanüle 404a. Die Figur 29 zeigt die Anordnung aus Figur 27 im Endzustand des Kanüleninsertionsmechanismus.

Wie bei der vorhergehend beschriebenen Ausführungsform wird der Kanüleninsertionsmechanismus bei dieser Ausführungsform durch einen Elektromotor angetrieben. In Unterschied zu Patch Infusionspumpe 300 wird der Kanüleninsertionsmechanismus bei Patch Infusionspumpe 400 nicht über eine Zahnstange angetrieben. Der Motor 411, welcher in der Patch Infusionspumpe 400, insbesondere am Gehäuse 402 oder der Basis 402a, befestigt ist (nicht gezeigt in den Figuren), treibt ein Kegelzahnrad 412 an, welches über seine Zahnung (nicht gezeigt) mit dem ebenfalls als Kegelzahnrad ausgebildeten Einleitrad 407e gekoppelt ist. Eine motorinduzierte Rotation des Kegelzahnrades 412 wird auf das Einleitrad 407e und somit auf das mit dem Einleitrad 407e festverbundene Zahnrad 407, welches seinerseits den Schlitten 403 in Bewegung versetzt, wodurch in der Folge die Infusionskanüle 405a zusammen mit der Insertionskanüle 404a ins Gewebe der benutzenden Person bewegt werden und anschliessend die Insertionskanüle 404a aus dem Gewebe zurück in die Patch Infusionspumpe 400, analog zu den vorher beschriebenen Ausführungsformen und Gestaltungen.

Bei dieser erfindungsgemässen Ausführungsform braucht es also keine Zahnstange mehr, wobei hier das Kegelzahnrad 412 die Funktion des Übertragungselementes übernimmt. Somit kann der Antrieb des Kanüleninsertionsmechanismus platzsparender gestaltet werden als bei der vorhergehenden Ausführungsform.

In einer alternativen Form der Patch Infusionspumpe 400 wird die Kegelzahnradverbindung zwischen den Teilen 412 und 407e durch eine Reibverbindung ersetzt. Der Motor 411 treibt in dieser alternativen Variante ein Kegelreibrad (analog zu Kegelzahnrad 412) an, welches in einer Reibverbindung zum Einleitrad 407e steht, welches in der Alternative ebenfalls als Kegelreibrad ausgebildet ist.

Der Elektromotorantrieb des Kanüleninsertionsmechanismus der Patch Infusionspumpe 400 hat ansonsten die gleichen Vorteile wie der zu Patch Infusionspumpe 300 beschriebene Elektromotorantrieb.

Die Elektromotoren 311 und 411 der Patch Infusionspumpen 300 respektive 400 können in ergänzenden Ausgestaltungen auch für die Verabreichung von fluider Substanz verwendet werden. Dies kann geschehen, in dem nach dem beendeten Rückzug der Insertionskanüle, also nachdem der Kanüleninsertionsprozess vollständig abgeschlossen ist, die Kopplung zwischen Motor und Einleitrad gelöst wird, so dass der Motor weiterdrehen kann, ohne den Kanüleninsertionsmechanismus weiter anzutreiben. Im Falle der Patch Infusionspumpe 300 kann das beispielhaft geschehen, indem dass die Zahnung 310a so dimensioniert ist, dass nach Abschluss des Insertionsprozesses auch das proximale Ende der Zahnung 310 erreicht ist. Wenn im Anschluss der Motor 311 in dieselbe Richtung weiter rotiert, verlässt das Zahnrad 312 die Zahnung 310a und kann somit frei weiter drehen, respektive folgend mit einem Verabreichungsantrieb für die Patch Infusionspumpe 300 koppeln. Bei Patch Infusionspumpe 400 kann das Auskoppel von Kegelzahnrad 412 und Einleitrad 407e geschehen, indem das Kegelzahnrad 412 entlang der Motorachse (nicht gezeigt) des Elektromotors 411 leicht vom Einleitrad 407e weg verschoben wird.

| Bezeichner | | | |
|---|---|---|---|
| | | 6 | Antriebsfeder |
| 1 | Patch Infusionspumpe | | |
| | | 7 | Zahnrad |
| 2 | Gehäuse | 7a | erster Sektor |

(fortgesetzt)

| | | | |
|---|---|---|---|
| 2a | Basis | 7b | zweiter Sektor |
| 2b | Führungsbahnen | 7c | Zähne |
| 2c | Federhalterung | 7d | Zahnradachse |
| 2d | Kanülenführung | | |
| 2e | trichterartige Führung | 8 | Freigabeklammer |
| 2f | Kanülenhalter | 8a | Federelemente |
| 2g | Freigabearme | 8b | Führungsarme |
| 2h | freies Ende mit Zahn | 8c | Bucht |
| 2i | Ausbuchtung | 8d | freies Ende |
| 2j | Führung | 8e | Blockierzone |
| 3 | Schlitten | 9 | Zuleitung |
| 3a | Führungskulisse | | |
| 3b | erste Teilkulisse | 100 | Patch Infusionspumpe |
| 3c | zweite Teilkulisse | | |
| 3d | Kanülenbrücke | 102 | Gehäuse |
| 3e | Führungselemente | 102a | Basis |
| 3f | Zahn | 102b | Führungsbahnen |
| 3g | Halbzahn | 102c | Zahnradlager |
| 3h | Bogen | 102d 102e | Kanülenführung trichterartige Führung |
| 4 | Insertionskanülenensemble | 102f | Kanülenhalter |
| 4a | Insertionskanüle | 102g | Druckfederwiderlager |
| 4b | Insertionskanülenträger | 102h 102k | Bohrung Zahnstangenführung |
| 5 | Kanülenensemble | 102l | stiftförmige Verlängerung |
| 5a | Infusionskanüle | | |
| 5b | Kanülenträger | 103 | Schlitten |
| 5c | Schnapparme | 103a | Führungskulisse |
| 5d | Haltearme | 103b | erste Teilkulisse |
| 5e | Führungsschlauch | 103c 103d | zweite Teilkulisse Kanülenbrücke |
| 103e | Führungselemente | 202 | Gehäuse |
| 103f | Zahn | 202a | Basis |
| 103g | Halbzahn | 202b | Führungsbahnen |
| 103h | Bogen | 202c | Zahnradlager |
| | | 202d | Kanülenführung |
| 104 | Insertionskanülenensemble | 202e | trichterartige Führung |
| 104a | Insertionskanüle | 202f | Kanülenhalter |
| 104b | Insertionskanülenträger | 202g | Federlager |
| | | 202k | Zahnstangenführung |
| 105 | Kanülenensemble | | |
| 105a | Infusionskanüle | 203 | Schlitten |
| 105b | Kanülenträger | 203a | Führungskulisse |
| 105c | Schnapparme | 203b | erste Teilkulisse |
| 105d | Haltearme | 203c | zweite Teilkulisse |
| 105e | Führungsschlauch | 203d | Kanülenbrücke |
| | | 203e | Führungselemente |
| 106 | Antriebsfeder | 203g | Halbzahn |
| | | 203h | Bogen |
| 107 | Zahnrad | | |
| 107a | erster Sektor | 204 | Insertionskanülenensemble |
| 107b | zweiter Sektor | 204a | Insertionskanüle |
| 107c | Zähne | 204b | Insertionskanülenträger |
| 107e | Einleitrad | | |

(fortgesetzt)

| | | | |
|---|---|---|---|
| | | 205 | Kanülenensemble |
| 108 | Freigabestift | 205a | Infusionskanüle |
| | | 205b | Kanülenträger |
| 109 | Zuleitung | 205c | Schnapparme |
| | | 205d | Haltearme |
| 110 | Zahnstange | 205e | Führungsschlauch |
| 110a | Zahnung | | |
| 110b | Federlager | 206 | Antriebsfeder |
| 110c | Führungselemente | | |
| 110d | Bohrung | 207 | Zahnrad |
| 110e | stiftförmige Verlängerung | 207a | erster Sektor |
| | | 207b | zweiter Sektor |
| 200 | Patch Infusionspumpe | 207c | Zähne |
| | | 207e | Einleitrad |
| | | 305e | Führungsschlauch |
| 209 | Zuleitung | | |
| | | 307 | Zahnrad |
| 210 | Zahnstange | 307a | erster Sektor |
| 210a | Zahnung | 307b | zweiter Sektor |
| 210b | Federlager | 307c | Zähne |
| 210c | Führungselemente | 307e | Einleitrad |
| 300 | Patch Infusionspumpe | 309 | Zuleitung |
| 302 | Gehäuse | 310 | Zahnstange |
| 302a | Basis | 310a | Zahnung |
| 302b | Führungsbahnen | 310c | Führungselemente |
| 302c | Zahnradlager | 310e | zweite Zahnung |
| 302d | Kanülenführung | | |
| 302e | trichterartige Führung | 311 | Elektromotor |
| 302f | Kanülenhalter | | |
| 302k | Zahnstangenführung | 312 | Zahnrad |
| 303 | Schlitten | 400 | Patch Infusionspumpe |
| 303a | Führungskulisse | | |
| 303b | erste Teilkulisse | 402 | Gehäuse |
| 303c | zweite Teilkulisse | 402a | Basis |
| 303d | Kanülenbrücke | 402b | Führungsbahnen |
| 303e | Führungselemente | 402c | Zahnradlager |
| 303g | Halbzahn | 402d | Kanülenführung |
| 303h | Bogen | 402e | trichterartige Führung |
| | | 402f | Kanülenhalter |
| 304 | Insertionskanülenensemble | | |
| 304a | Insertionskanüle | 403 | Schlitten |
| 304b | Insertionskanülenträger | 403a | Führungskulisse |
| | | 403b | erste Teilkulisse |
| 305 | Kanülenensemble | 403c | zweite Teilkulisse |
| 305a | Infusionskanüle | 403d | Kanülenbrücke |
| 305b | Kanülenträger | 403e | Führungselemente |
| 305c | Schnapparme | 403g | Halbzahn |
| 305d | Haltearme | 403h | Bogen |
| | | 407 | Zahnrad |
| 404 | Insertionskanülenensemble | 407a | erster Sektor |
| 404a | Insertionskanüle | 407b | zweiter Sektor |

(fortgesetzt)

| | | | |
|---|---|---|---|
| 404b | Insertionskanülenträger | 407c | Zähne |
| | | 407e | Einleitrad |
| 405 | Kanülenensemble | | |
| 405a | Infusionskanüle | 409 | Zuleitung |
| 405b | Kanülenträger | | |
| 405c | Schnapparme | 411 | Elektromotor |
| 405d | Haltearme | | |
| 405e | Führungsschlauch | 412 | Kegelrad |

**Patentansprüche**

1.  Ein Antrieb für einen Insertionsmechanismus für eine Verabreichungsvorrichtung (100) aufweisend

    - eine ebene oder gewölbte Basis (102a)
    - eine in oder auf der Basis (102a) gebildete Führungsbahn (102b), welche ein proximales und ein distales Ende und dazwischen eine Gerade oder eine Kurve definiert
    - einen Schlitten (103) rotationsfest an der Basis (102a) vorgesehen und entlang oder in der Führungsbahn (102b) zwischen einer proximalen Schlittenendposition und einer distalen Schlittenendposition verschiebbar, insbesondere hin und her bewegbar,
    - ein Antriebsrad drehbar an der Basis gelagert, welches als Zahnrad (107) ausgebildet ist und zwei Sektoren (107a, 107b) aufweist, wobei in einem Sektor (107a) der Umfang des Zahnrades gezahnt (107c) ist und im anderen Sektor (107b) keine Zähne vorhanden sind ,
    - eine mit dem Schlitten (103) verbundene Antriebsbahn, welche als Führungskulisse (103a) ausgebildet ist, aufweisend mehrere Antriebsabschnitte, welche als Teilkulissen (103b, 103c) ausgebildet sind und welche Zahnungen (103f) oder Stiftanordnungen zum Eingriff in die Zahnung (107c) des Antriebsrades umfassen, wobei ein erster Antriebsabschnitt (103b) und ein zweiter Antriebsabschnitt (103c) sich gegenüberliegen und zur Gerade oder zur Kurve der Führungsbahn (102b) parallel verlaufen,
    - eine Energiequelle (106), durch welche das Antriebsrad relativ zu Basis und Schlitten in Rotation versetzbar ist, wobei bei Rotation des Antriebsrades (107) der Schlitten (103) ausgehend von insbesondere der proximalen Schlittenendposition durch einen form- oder kraftschlüssigen Eingriff des gezahnten Sektors (107a) des Antriebsrads (107) in den ersten Antriebsabschnitt entlang oder in der Führungsbahn in distale Richtung verschiebt, bis am distalen Ende des ersten Antriebsabschnitts der form- oder kraftschlüssigen Eingriff des gezahnten Sektors (107a) des Antriebsrads vom ersten auf den zweiten Antriebsabschnitt durch ein Weiterdrehen des Antriebsrads (107) wechselt und der Schlitten (103) folglich entlang oder in der Führungsbahn (102b) in proximale Richtung verschiebt
    ***dadurch gekennzeichnet*, dass**
    die Energiequelle (106) über ein ein- oder mehrteiliges Übertragungselement (110) und ein Einleitrad (107e) mit dem Antriebsrad (107) koppelbar ist, wobei das Einleitrad (107e) verdrehgesichert mit dem Antriebsrad verbunden ist, und wobei das Übertragungselement (110) verschiebbar oder rotierbar zur Basis angeordnet ist.

2.  Ein Antrieb nach Anspruch 1, wobei das Übertragungselement eine Zahnstange (110) mit mindestens einer Zahnung (110a) ist und verschiebbar an der Basis (102a) angeordnet ist und wobei das Einleitrad (107e) ein Zahnrad ist, dessen Zähne in Eingriff mit einer Zahnung (110a) der Zahnstange (110) bringbar sind.

3.  Ein Antrieb nach dem vorhergehenden Anspruch, wobei die Basis (102a) weiter eine lineare Zahnstangenführung (102k) umfasst, insbesondere in Form einer Nut, und wobei die Zahnstange (110) mindestens ein Führungselement (110c) umfasst, insbesondere mindestens zwei Nocken, über welches die Zahnstange (110) mit der Basis (102a) verbunden ist und wobei die Zahnstange (110) entlang der Zahnstangenführung (102k) verschiebbar ist.

4.  Ein Antrieb nach dem vorhergehenden Anspruch, wobei die Energiequelle (106) eine Zug- oder Druckfeder ist, wobei ein Ende der Zug- oder Druckfeder fest zur Basis (102a) angeordnet ist und zweites Ende fest an der Zahnstange (110) angeordnet ist und wobei die Zug- oder Druckfeder für den Antrieb des Antriebs für den Insertionsmechanismus der Verabreichungsvorrichtung vorgespannt oder vorspannbar ist und wobei durch eine Freigabe der vorgespannten Zug- oder Druckfeder eine Kraft auf die Zahnstange (110) wirkt, welche diese entlang der Zahnstangenführung (102k)in Bewegung versetzt und wodurch in der Folge Einleit- (107e) und Antriebsrad (107) in eine Rotation versetzt werden.

5. Ein Antrieb nach einem der Ansprüche 2 bis 4, wobei der Antrieb weiter eine Freigabevorrichtung umfasst, wobei die Freigabevorrichtung mindestens folgende Elemente umfasst,

- ein fest an der Basis angeordnetes Halteelement (102h),
- ein fest an der Zahnstange angeordnetes weiteres Halteelement(110d), und
- ein Verbindungselement (108), mit welchem das Halteelement und das weitere Haltelement lösbar miteinander verbindbar sind,

so dass, wenn das Halteelement (102h) und das weitere Halteelement (110d) über das Verbindungselement (108) miteinander verbunden sind, die Zahnstange (110) relativ zur Basis fest gehalten oder fixiert wird.

6. Ein Antrieb nach dem vorhergehenden Anspruch, wobei es sich beim Halteelement (102h) und dem weiteren Halteelement (110d) um Bohrungen handelt und das Verbindungselement (108) als Stift oder Splint ausgebildet ist, welcher in die Bohrungen einführbar ist und dadurch die Zahnstange (110) an der Basis (102a) fixierbar ist

7. Ein Antrieb nach Anspruch 2, wobei die Zahnstange (310) eine erste (310a) und eine zweite Zahnung (310e) aufweist, wobei die erste Zahnung (310a) in Eingriff mit dem als Zahnrad ausgebildeten Einleitrad (307e) bringbar ist und wobei die Energiequelle (311) über die zweite Zahnung (310e) mit der Zahnstange (310) kuppelbar ist.

8. Ein Antrieb nach dem vorhergehenden Anspruch, wobei die Energiequelle ein Elektromotor (311)mit einer Elektromotorantriebsachse, welche durch den Elektromotor direkt oder über ein Getriebe in Rotation versetzbar ist, und wobei an der Elektromotorantriebsachse koaxial und fest ein Zahnrad (312) angeordnet ist, welche in Eingriff mit der zweiten Zahnung (310e) der Zahnstange (310) bringbar ist.

9. Ein Antrieb nach Anspruch 1, wobei das Einleitrad als eine erstes Kegelrad (407e), insbesondere als Kegelzahnrad, ausgeführt ist und das Übertragungselement ebenfalls als ein zweites Kegelrad (412), insbesondere als Kegelzahnrad ausgeführt ist, wobei erstes Kegelrad (407e) und zweites Kegelrad (412) miteinander in Eingriff stehen, so dass eine Rotation des zweiten Kegelrades (412) eine Rotation des ersten Kegelrades (407e) bewirkt und wobei die Rotationsachsen des ersten und des zweiten Kegelrades in einem Winkel von etwa 90° zueinander stehen.

10. Ein Antrieb nach dem vorhergehenden Anspruch, wobei das zweite Kegelrad durch einen elektrischen Antrieb (411) oder einer Feder in Rotation versetzbar ist.

11. Ein Antrieb nach einem der vorhergehenden Ansprüche, wobei am Schlitten (3) eine Zuleitung (9) mit einem Lumen angeordnet und in oder am Schlitten (3) geführt ist, durch welche eine zu verabreichende Substanz, insbesondere ein fluides Medikament, geleitet werden kann.

12. Ein Antrieb nach dem vorhergehenden Anspruch, wobei am distalen Ende des Schlittens eine Kanülenbrücke (3d) angeordnet ist, an welcher die Zuleitung (9) endet und ein proximales Ende einer Insertionskanüle (4a) mit einem Lumen angeordnet ist, wobei das Lumen der Zuleitung und das Lumen der Insertionskanüle (4a) miteinander verbunden sind, so dass die zu verabreichende Substanz von der Zuleitung in die Insertionskanüle (4a) geleitet werden kann, und wobei die Insertionskanüle (4a) eine Verschiebung des Schlittens (3) in distale wie in proximale Richtung mitmacht.

13. Ein Kanüleninsertionsmechanismus für ein Patch Gerät, umfassend

- einen Antrieb nach dem vorhergehenden Anspruch,
- eine Infusionskanüle (5a) mit einem distalen und einem proximalen Ende, durch welche die Insertionskanüle (4a) hindurchführbar ist,

wobei am proximalen Ende der Infusionskanüle ein Kanülenträger (5b) fest angeordnet ist, wobei am Kanülenträger (5b) ein Verbindungmittel, insbesondere ein oder mehrere Schnapparme (5c), angeordnet ist, über welches der Kanülenträger (5b) lösbar mit der Kanülenbrücke (3d)verbindbar ist, wenn die Insertionskanüle (4a) soweit durch die Infusionskanüle hindurch geführt ist, dass der Kanülenträger (5b) an der Kanülenbrücke (3d) anliegt.

**14.** Ein Patch Gerät, insbesondere eine Patch Pumpe (1) oder ein Patch Injektionsgerät, mit einem Kanüleninsertionsmechanismus nach dem vorhergehenden Anspruch.

**Claims**

**1.** A drive for an insertion mechanism for an administering device (100) having

- a planar or curved base (102a)
- a guide track (102b) which is formed in or on the base (102a) and defines a proximal and a distal end and a straight line or a curve therebetween
- a slide (103) which is provided in a rotationally fixed manner on the base (102a) and is slidable, in particular is movable back and forth, along or in the guide track (102b) between a proximal slide end position and a distal slide end position,
- a drive wheel rotatably mounted on the base, which wheel is designed as a pinion (107) and has two sectors (107a, 107b), the circumference of the pinion being toothed (107c) in one sector (107a) and no teeth being present in the other sector (107b),
- a drive track which is connected to the slide (103) and is designed as a guide slot (103a), the drive track having a plurality of drive portions which are designed as partial slots (103b, 103c) and which comprise toothings (103f) or pin arrangements for engagement in the toothing (107c) of the drive wheel, a first drive portion (103b) and a second drive portion (103c) being opposite one another and extending in parallel with the straight line or with the curve of the guide track (102b),
- an energy source (106) by means of which the drive wheel can be rotated relative to the base and the slide,

upon rotation of the drive wheel (107), the slide (103) sliding, starting from in particular the proximal slide end position, by interlocking or frictional engagement of the toothed sector (107a) of the drive wheel (107) in the first drive portion, along or in the guide track in the distal direction, until, at the distal end of the first drive portion, the interlocking or frictional engagement of the toothed sector (107a) of the drive wheel switches from the first to the second drive portion by a further rotation of the drive wheel (107), and the slide (103) consequently slides along or in the guide track (102b) in the proximal direction,
**characterized in that**
the energy source (106) can be coupled to the drive wheel (107) via a one-part or multi-part transmission element (110) and an introduction wheel (107e), the introduction wheel (107e) being connected to the drive wheel so as to be secured against rotation, and the transmission element (110) being arranged slidably or rotatably with respect to the base.

**2.** A drive according to claim 1, wherein the transmission element is a rack (110) comprising at least one toothing (110a) and is slidably arranged on the base (102a), and wherein the introduction wheel (107e) is a pinion, the teeth of which can be brought into engagement with a toothing (110a) of the rack (110).

**3.** A drive according to the preceding claim, wherein the base (102a) further comprises a linear rack guide (102k), in particular in the form of a groove, and wherein the rack (110) comprises at least one guide element (110c), in particular at least two cams, via which the rack (110) is connected to the base (102a), and wherein the rack (110) is slidable along the rack guide (102k).

**4.** A drive according to the preceding claim, wherein the energy source (106) is a tension spring or compression spring, wherein one end of the tension spring or compression spring is fixedly arranged with respect to the base (102a) and the second end is fixedly arranged on the rack (110), and wherein the tension spring or compression spring is preloaded or can be preloaded for the driving of the drive for the insertion mechanism of the administering device, and wherein due to a release of the preloaded tension spring or compression spring, a force acts on the rack (110), which force causes said rack to move along the rack guide (102k), and the introduction wheel (107e) and the drive wheel (107) are thereby subsequently rotated.

**5.** A drive according to any of claims 2 to 4, wherein the drive further comprises a release device, wherein the release device comprises at least the following elements,

• a holding element (102h) arranged fixedly on the base,
• a further holding element (110d) arranged fixedly on the rack, and

• a connecting element (108) by means of which the holding element and the further holding element are detachably interconnectable,

such that when the holding element (102h) and the further holding element (110d) are interconnected via the connecting element (108), the rack (110) is fixedly held or fastened relative to the base.

6. A drive according to the preceding claim, wherein the holding element (102h) and the further holding element (110d) are holes and the connecting element (108) is designed as a pin or a cotter pin which can be inserted into the holes and thus can fasten the rack (110) to the base (102a)

7. A drive according to claim 2, wherein the rack (310) has a first toothing (310a) and a second toothing (310e), wherein the first toothing (310a) can be brought into engagement with the introduction wheel (307e) designed as a pinion, and wherein the energy source (311) can be coupled to the rack (310) via the second toothing (310e).

8. A drive according to the preceding claim, wherein the energy source is an electric motor (311) comprising an electric motor drive axle which can be rotated by the electric motor directly or via a transmission, and wherein a pinion (312) is arranged coaxially and fixedly on the electric motor drive axle and can be brought into engagement with the second toothing (310e) of the rack (310).

9. A drive according to claim 1, wherein the introduction wheel is designed as a first bevel gear (407e), in particular as a bevel pinion, and the transmission element is likewise designed as a second bevel gear (412), in particular as a bevel pinion, wherein the first bevel gear (407e) and the second bevel gear (412) are in engagement with one another such that a rotation of the second bevel gear (412) causes a rotation of the first bevel gear (407e), and wherein the axes of rotation of the first and the second bevel gear are at an angle of approximately 90° to one another.

10. A drive according to the preceding claim, wherein the second bevel gear can be rotated by an electric drive (411) or a spring.

11. A drive according to any of the preceding claims, wherein a feed line (9) having a lumen is arranged on the slide (3) and is guided in or on the slide (3), through which feed line a substance to be administered, in particular a fluid medicament, can be conducted.

12. A drive according to the preceding claim, wherein a cannula bridge (3d) is arranged at the distal end of the slide, at which bridge the feed line (9) ends and a proximal end of an insertion cannula (4a) comprising a lumen is arranged, wherein the lumen of the feed line and the lumen of the insertion cannula (4a) are interconnected such that the substance to be administered can be conducted from the feed line into the insertion cannula (4a), and wherein the insertion cannula (4a) slides along with the slide (3) in the distal direction and in the proximal direction.

13. A cannula insertion mechanism for a patch device, comprising

- a drive according to the preceding claim,
- an infusion cannula (5a) having a distal end and a proximal end, through which the insertion cannula (4a) can be guided,

wherein a cannula support (5b) is fixedly arranged at the proximal end of the infusion cannula, wherein a connection means, in particular one or more snapon arms (5c), is arranged on the cannula support (5b), via which connection means the cannula support (5b) can be detachably connected to the cannula bridge (3d) when the insertion cannula (4a) is guided through the infusion cannula so far that the cannula support (5b) rests against the cannula bridge (3d).

14. A patch device, in particular a patch pump (1) or a patch injection device, comprising a cannula insertion mechanism according to the preceding claim.

**Revendications**

1. Entraînement pour un mécanisme d'insertion destiné à un dispositif d'administration (100), présentant

- une base plane ou bombée (102a)

- une voie de guidage (102b) formée dans ou sur la base (102a), laquelle voie de guidage définit une extrémité proximale et une extrémité distale et une ligne droite ou une courbe entre celles-ci
- une glissière (103) prévue de manière fixe en rotation au niveau de la base (102a) et pouvant coulisser, en particulier pouvant se déplacer en va-et-vient, le long de la voie de guidage (102b) ou dans celle-ci entre une position d'extrémité de glissière proximale et une position d'extrémité de glissière distale,
- une roue d'entraînement montée de manière à pouvoir pivoter au niveau de la base, laquelle roue d'entraînement est réalisée sous forme d'une roue dentée (107) et présente deux secteurs (107a, 107b), dans lequel la circonférence de la roue dentée est dentée (107c) dans un secteur (107a) et aucune dent n'est présente dans l'autre secteur (107b),
- une voie d'entraînement reliée à la glissière (103), laquelle voie d'entraînement est réalisée sous la forme d'une coulisse de guidage (103a), présentant plusieurs sections d'entraînement qui sont réalisées sous forme de coulisses partielles (103b, 103c) et qui comprennent des dentures (103f) ou des agencements de broches pour la mise en prise dans la denture (107c) de la roue d'entraînement, dans lequel une première section d'entraînement (103b) et une seconde section d'entraînement (103c) sont opposées l'une à l'autre et s'étendent parallèlement à la ligne droite ou à la courbe de la voie de guidage (102b),
- une source d'énergie (106) par laquelle la roue d'entraînement peut être mise en rotation par rapport à la base et à la glissière,

dans lequel, lors de la rotation de la roue d'entraînement (107), la glissière (103) coulisse dans la direction distale le long de la voie de guidage ou dans celle-ci, à partir en particulier de la position d'extrémité de glissière proximale, grâce à une mise en prise par complémentarité de forme ou à force du secteur denté (107a) de la roue d'entraînement (107) dans la première section d'entraînement, jusqu'à ce que, au niveau de l'extrémité distale de la première section d'entraînement, la mise en prise par complémentarité de forme ou à force du secteur denté (107a) de la roue d'entraînement passe de la première section d'entraînement à la seconde section d'entraînement par une poursuite de la rotation de la roue d'entraînement (107) et que la glissière (103) coulisse par conséquent le long de la voie de guidage (102b) ou dans celle-ci dans la direction proximale
**caractérisé en ce que**
la source d'énergie (106) peut être accouplée à la roue d'entraînement (107) par l'intermédiaire d'un élément de transmission (110) à une ou plusieurs parties et d'une roue d'introduction (107e), dans lequel la roue d'introduction (107e) est reliée à la roue d'entraînement de manière à être bloquée en rotation, et dans lequel l'élément de transmission (110) est disposé de manière à pouvoir coulisser ou à pouvoir tourner par rapport à la base.

2. Entraînement selon la revendication 1, dans lequel l'élément de transmission est une crémaillère (110) comportant au moins une denture (110a) et est disposé de manière à pouvoir coulisser au niveau de la base (102a), et dans lequel la roue d'introduction (107e) est une roue dentée dont les dents peuvent être amenées en prise avec une denture (110a) de la crémaillère (110).

3. Entraînement selon la revendication précédente, dans lequel la base (102a) comprend en outre un guide de crémaillère (102k) linéaire, en particulier sous la forme d'une rainure, et dans lequel la crémaillère (110) comprend au moins un élément de guidage (110c), en particulier au moins deux cames, par l'intermédiaire duquel la crémaillère (110) est reliée à la base (102a), et dans lequel la crémaillère (110) peut coulisser le long du guide de crémaillère (102k).

4. Entraînement selon la revendication précédente, dans lequel la source d'énergie (106) est un ressort de traction ou de compression, dans lequel une extrémité du ressort de traction ou de compression est disposée de manière fixe par rapport à la base (102a) et une seconde extrémité est disposée de manière fixe au niveau de la crémaillère (110), et dans lequel le ressort de traction ou de compression est précontraint ou peut être précontraint pour entraîner l'entraînement pour le mécanisme d'insertion du dispositif d'administration, et dans lequel une force agit sur la crémaillère (110) grâce à une libération du ressort de traction ou de compression précontraint, laquelle force met la crémaillère en mouvement le long du guide de crémaillère (102k) et, par la suite, met en rotation la roue d'introduction (107e) et la roue d'entraînement (107).

5. Entraînement selon l'une des revendications 2 à 4, dans lequel l'entraînement comprend en outre un dispositif de libération, dans lequel le dispositif de libération comprend au moins les éléments suivants,

• un élément de retenue (102h) disposé de manière fixe au niveau de la base,

• un autre élément de retenue (110d) disposé de manière fixe au niveau de la crémaillère, et
• un élément de liaison (108) grâce auquel l'élément de retenue et l'autre élément de retenue peuvent être reliés l'un à l'autre de manière amovible,

de sorte que, lorsque l'élément de retenue (102h) et l'autre élément de retenue (110d) sont reliés l'un à l'autre par l'intermédiaire de l'élément de liaison (108), la crémaillère (110) est retenue de manière fixe par rapport à la base ou fixée sur celle-ci.

6. Entraînement selon la revendication précédente, dans lequel l'élément de retenue (102h) et l'autre élément de retenue (110d) sont des alésages et l'élément de liaison (108) est réalisé sous la forme d'une broche ou d'une goupille qui peut être insérée dans les alésages et qui permet ainsi de fixer la crémaillère (110) à la base (102a)

7. Entraînement selon la revendication 2, dans lequel la crémaillère (310) présente une première (310a) et une seconde denture (310e), dans lequel la première denture (310a) peut être amenée en prise avec la roue d'introduction (307e) réalisée sous la forme d'une roue dentée et dans lequel la source d'énergie (311) peut être accouplée à la crémaillère (310) par l'intermédiaire de la seconde denture (310e).

8. Entraînement selon la revendication précédente, dans lequel la source d'énergie est un moteur électrique (311) comportant un axe d'entraînement de moteur électrique qui peut être mis en rotation directement par le moteur électrique ou par l'intermédiaire d'un engrenage, et dans lequel une roue dentée (312) est disposée de manière coaxiale et fixe au niveau de l'axe d'entraînement de moteur électrique, laquelle roue dentée peut être amenée en prise avec la seconde denture (310e) de la crémaillère (310).

9. Entraînement selon la revendication 1, dans lequel la roue d'introduction est réalisée sous la forme d'une première roue conique (407e), en particulier d'une roue dentée conique, et l'élément de transmission est également réalisé sous la forme d'une seconde roue conique (412), en particulier d'une roue dentée conique, dans lequel la première roue conique (407e) et la seconde roue conique (412) sont en prise l'une avec l'autre, de sorte qu'une rotation de la seconde roue conique (412) provoque une rotation de la première roue conique (407e), et dans lequel les axes de rotation de la première et de la seconde roue conique forment un angle d'environ 90° l'un par rapport à l'autre.

10. Entraînement selon la revendication précédente, dans lequel la seconde roue conique peut être mise en rotation par un entraînement électrique (411) ou par un ressort.

11. Entraînement selon l'une des revendications précédentes, dans lequel une conduite d'alimentation (9) comportant une lumière est disposée au niveau de la glissière (3) et est guidée dans ou sur la glissière (3), à travers laquelle conduite d'alimentation une substance à administrer, en particulier un médicament fluide, peut être guidée.

12. Entraînement selon la revendication précédente, dans lequel un pont de canules (3d) est disposé au niveau de l'extrémité distale de la glissière, au niveau duquel pont de canules se termine la conduite d'alimentation (9) et est disposée une extrémité proximale d'une canule d'insertion (4a) comportant une lumière, dans lequel la lumière de la conduite d'alimentation et la lumière de la canule d'insertion (4a) sont reliées l'une à l'autre, de sorte que la substance à administrer peut être conduite depuis la conduite d'alimentation dans la canule d'insertion (4a), et dans lequel la canule d'insertion (4a) accompagne un coulissement de la glissière (3) dans la direction distale ainsi que dans la direction proximale.

13. Mécanisme d'insertion de canules pour un appareil de type patch, comprenant

- un entraînement selon la revendication précédente,
- une canule de perfusion (5a) comportant une extrémité distale et une extrémité proximale à travers lesquelles la canule d'insertion (4a) peut passer,

dans lequel un support de canules (5b) est disposé de manière fixe au niveau de l'extrémité proximale de la canule de perfusion, dans lequel un moyen de liaison, en particulier un ou plusieurs bras d'encliquetage (5c), est disposé au niveau du support de canules (5b), par l'intermédiaire duquel moyen de liaison le support de canules (5b) peut être relié de manière amovible au pont de canules (3d) lorsque la canule d'insertion (4a) est guidée à travers la canule de perfusion jusqu'à ce que le support de canules (5b) repose contre le pont de canules (3d).

14. Appareil de type patch, en particulier pompe de type patch (1) ou appareil d'injection de type patch, comportant un mécanisme d'insertion de canules selon la revendication précédente.

Fig. 2

Fig. 1

EP 3 570 908 B1

Fig. 4

Fig. 3a

Fig. 3b

24

Fig. 6

Fig. 5

Fig. 8

Fig. 7

Fig. 10

Fig. 9

Fig. 12

Fig. 11

EP 3 570 908 B1

Fig. 13a

Fig. 13b

Fig. 14a

Fig. 14b

Fig. 17

Fig. 15

Fig. 16

Fig. 19

Fig. 18

Fig. 21

Fig. 20

Fig. 23

Fig. 22

302b

312

311

310e

302k

305e

Fig. 24

302a

303g

303h

310a

305a

302d

302f

303g

304a

302e

Fig. 25

Fig. 27

Fig. 26

Fig. 29

Fig. 28

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 7128727 B2
- US 2014142508 A1

- WO 2016145094 A2